# EUROPEAN PATENT SPECIFICATION

(11) **EP 0 884 314 B1**
(45) Date of publication and mention of the grant of the patent: **21.01.2004**
(21) Application number: 98114788.7
(22) Date of filing: 22.09.1992
(51) Int. Cl.: C07D 305/14

(54) **Metal alkoxides**
Metallalkoxide
Alkoxydes de métaux

(30) Priority: 23.09.1991 US 763805; 03.04.1992 US 862955; 06.04.1992 US 863840
(43) Date of publication of application: 16.12.1998
(62) Divisional of application: 92921316.3
(73) Proprietor: FLORIDA STATE UNIVERSITY, Tallahassee, Florida 32310 (US)
(72) Inventor: Holton, Robert A., Florida State University, Room 715, Tallahassee, Florida 32306 (US)
(74) Representative: W.P. THOMPSON & CO.

(56) References cited:
- J.-N. DENIS, ET AL.: "A highly efficient, practical approach to natural taxol" JOURNAL OF THE AMERICAN CHEMICAL SOCIETY, vol. 110, no. 17, 17 August 1988 (1988-08-17), pages 5917-5919, XP000654163 American Chemical Society, Washington, DC, US ISSN: 0002-7863

## Description

### BACKGROUND OF THE INVENTION

Three esters of N-acyl phenyl isoserine, taxol, taxotere and cephalomannine have been found to possess significant properties as antitumor agents. This application describes a process for the preparation of N-acyl, N-sulfonyl and N-phosphoryl substituted isoserine esters, in general and to a semi-synthesis for the preparation of taxane derivatives such as taxol, taxotere and other biologically active derivatives involving the use of metal alkoxides and β-lactams, in particular.

The taxane family of terpenes, of which taxol is a member, has attracted considerable interest in both the biological and chemical arts. Taxol is a promising cancer chemotherapeutic agent with a broad spectrum of antileukemic and tumor-inhibiting activity. Taxol has the following structure: 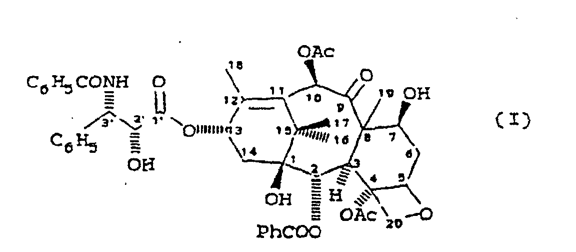 wherein Ph is phenyl and Ac is acetyl. Because of this promising activity, taxol is currently undergoing clinical trials in both France and the United States.

The supply of taxol for these clinical trials is presently being provided by the bark from Taxus brevifollia (Western Yew). However, taxol is found only in minute quantities in the bark of these slow growing evergreens, causing considerable concern that the limited supply of taxol will not meet the demand. Consequently, chemists in recent years have expended their energies in trying to find a viable synthetic route for the preparation of taxol. So far, the results have not been entirely satisfactory.

One synthetic route that has been proposed is directed to the synthesis of the tetracyclic taxane nucleus from commodity chemicals. A synthesis of the taxol congener taxusin has been reported by Holton, et al. in JACS 110, 6558 (1988). Despite the progress made in this approach, the final total synthesis of taxol is, nevertheless, likely to be a multi-step, tedious, and costly process.

A semi-synthetic approach to the preparation of taxol has been described by Greene, et al. in JACS 110, 5917 (1988), and involves the use of a congener of taxol, 10-deacetyl baccatin III which has the structure of formula II shown below: 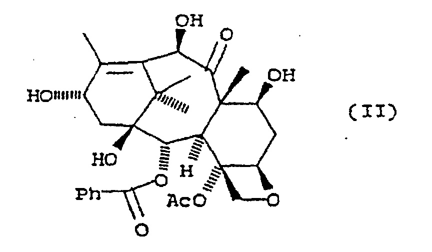 10-deacetyl baccatin III is more readily available than taxol since it can be obtained from the needles of Taxus baccata. According to the method of Greene et al., 10-deacetyl baccatin III is converted to taxol by attachment of the C-10 acetyl group and by attachment of the C-13 β-amido ester side chain through the esterification of the C-13 alcohol with a β-amido carboxylic acid unit. Although this approach requires relatively few steps, the synthesis of the β-amido carboxylic acid unit is a multi-step process which proceeds in low yield, and the coupling reaction is tedious and also proceeds in low yield. However, this coupling reaction is a key step which is required in every contemplated synthesis of taxol or biologically active derivative of taxol, since it has been shown by Wani, et al. in JACS 93, 2325 (1971) that the presence of the β-amido ester side chain at C13 is required for anti-tumor activity.

More recently, it has been reported in Colin et al. U.S. Patent No. 4,814,470 that taxol derivatives of the formula III below, have an activity significantly greater than that of taxol (I). 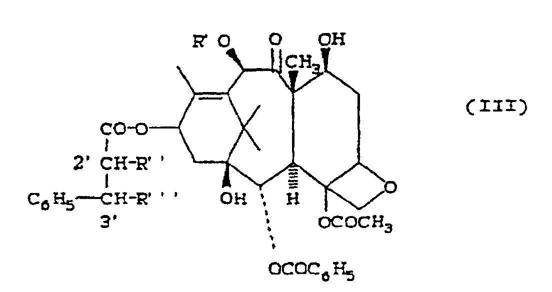 R' represents hydrogen or acetyl and one of R" and R"' represents hydroxy and the other represents tert-butoxy-carbonylamino and their stereoisomeric forms, and mixtures thereof.

According to Colin et al., U.S. Patent 4,418,470, the products of general formula (III) are obtained by the action of the sodium salt of tert-butyl N-chlorocarbamate on a product of general formula: 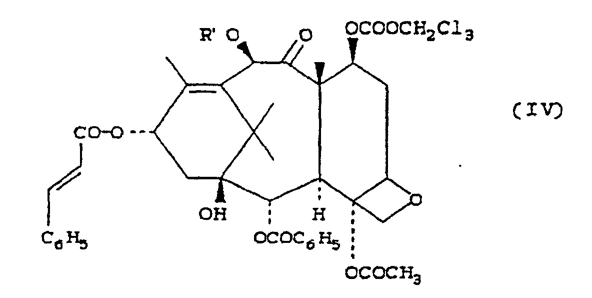 in which R' denotes an acetyl or 2,2,2-trichloroethoxycarbonyl radical, followed by the replacement of the 2,2,2-trichloroethoxycarbonyl group or groups by hydrogen. It is reported by Denis et al. in U.S. Patent No. 4,924,011, however, that this process leads to a mixture of isomers which has to be separated and, as a result, not all the baccatin III or 10-deactylbaccatin III employed for the preparation of the product of general formula (IV) can be converted to a product of general formula (III).

In an effort to improve upon the Colin et al. process, Denis et al. disclose a different process for preparing derivatives of baccatin III or of 10-deactylbaccatin III of general formula 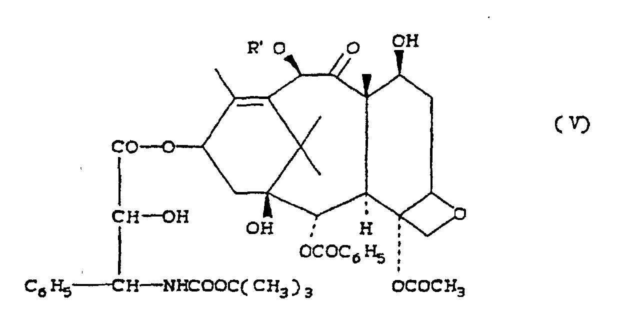 in which R' denotes hydrogen or acetyl wherein an acid of general formula: 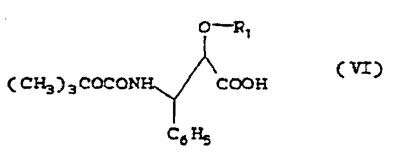 in which R₁ is a hydroxy-protecting group, is condensed with a taxane derivative of general formula: 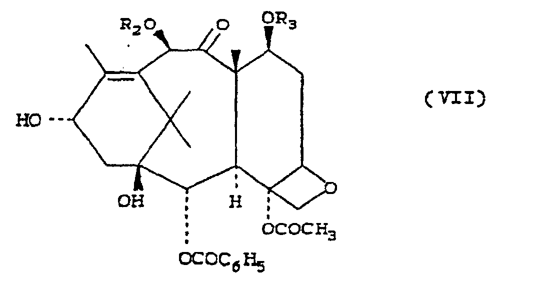 in which R₂ is an acetyl hydroxy-protecting group and R₃ is a hydroxy-protecting group, and the protecting groups R₁, R₃ and, where appropriate, R₂ are then replaced by hydrogen. However, this method employs relatively harsh conditions, proceeds with poor conversion, and provides less than optimal yields.

A major difficulty remaining in the synthesis of taxol and other potential anti-tumor agents is the lack of a readily available method for easy attachment, to the C-13 oxygen, of the chemical unit which provides the ß-amido ester side chain. Development of such a process for its attachment in high yield would facilitate the synthesis of taxol as well as related anti-tumor agents having a modified set of nuclear substituents or a modified C-13 side chain. This need has been fulfilled by the discovery of a new, efficient process for attachment, to the C-13 oxygen, of the chemical unit which provides the ß-amido ester side chain.

Another major difficulty encountered in the synthesis of taxol is that known processes for the attachment of the β-amido ester side chain at C-13 are generally not sufficiently diastereoselective. Therefore the side chain precursor must be prepared in optically active form to obtain the desired diastereomer during attachment. The process described herein, however, is highly diastereoselective, thus permitting the use of a racemic mixture of side chain precursor, eliminating the need for the expensive, time-consuming process of separating the precursor into its respective enantiomeric forms. The reaction additionally proceeds at a faster rate than previous processes, thus permitting the use of less side-chain precursor than has been required by such previous processes.

### SUMMARY OF THE INVENTION

A process is described herein for the preparation of N-acyl, N-sulfonyl and N-phosphoryl esters of isoserine. Also described are a side chain precursor for the synthesis of taxane derivatives; a process for the attachment of the side chain precursor in relatively high yield to provide an intermediate which is readily converted to the desired taxane derivative; and such a process which is highly diastereo-selective.

In accordance with the present invention, there is provided a metal alkoxide having the formula: 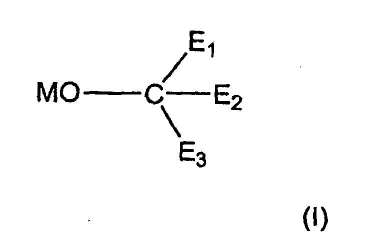 wherein
M is a metal;
E₁, E₂ and the carbon to which they are attached comprise a bi-, tri- or tetracyclic taxane nucleus, and
E₃ is hydrogen.

According to a preferred aspect there is provided a metal alkoxide having the formula: 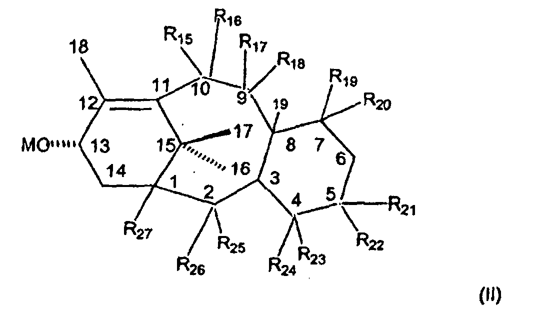 wherein
M is a metal;
R₁₅ and R₁₆ are independently hydrogen, hydroxy, lower alkanoyloxy, alkenoyloxy, alkynoyloxy, aryloyloxy or R₁₅ and R₁₆ together form an oxo;
R₁₇ and R₁₈ are independently hydrogen or lower alkanoyloxy, alkenoyloxy, alkynoyloxy, or aryloyloxy or R₁₇ and R₁₈ together form an oxo;
R₁₉ and R₂₀ are independently hydrogen or hydroxy or lower alkanoyloxy, alkenoyloxy, alkynoyloxy, or aryloyloxy;
R₂₁ and R₂₂ are independently hydrogen or lower alkanoyloxy, alkenoyloxy, alkynoyloxy, or aryloyloxy or R₂₁ and R₂₂ together form an oxo;
R₂₃ and R₂₄ are independently hydrogen or hydroxy or lower alkanoyloxy, alkenoyloxy, alkynoyloxy, or aryloyloxy or
R₂₃ and R₂₄ together form an oxo or methylene or
R₂₃ and R₂₄ together with the carbon atom to which they are attached form an oxirane ring or
R₂₃ and R₂₂ together with the carbon atom to which they are attached form an oxetane ring;
R₂₅ is hydrogen, hydroxy, or lower alkanoyloxy, alkenoyloxy, alkynoyloxy, or aryloyloxy;
R₂₆ is hydrogen, hydroxy, or lower alkanoyloxy, alkenoyloxy, alkynoyloxy, or aryloyloxy; or
R₂₅ and R₂₆ taken together form an oxo; and
R₂₇ is hydrogen, hydroxy or lower alkoxy, alkanoyloxy, alkenoyloxy, alkynoyloxy, or aryloyloxy.

In a metal alkoxide of formula (II) R₂₂ and R₂₃ together with the carbon atoms to which they are attached may form an oxetane ring.

In a preferred metal alkoxide of formula (II):
R₁₅ is -OT₂ or -OCOCH₃;
R₁₆ is hydrogen;
R₁₇ and R₁₈ together form an oxo;
R₁₉ is -OT₁;
R₂₀ and R₂₁ are hydrogen;
R₂₂ and R₂₃ and the carbons to which they are attached form an oxetane ring;
R₂₄ is - OCOCH₃
R₂₅ is PhCOO-;
R₂₆ is hydrogen;
R₂₇ is hydroxy; and
T₁ and T₂ are independently hydrogen or hydroxy protecting groups.

Also described herein is a process for the preparation of isoserine esters having the formula M may be a Group IA, IIA, IIIA, lanthanide, actinide, or a transition Group IIIA, IVA, VA, or VIA metal. For example, M lithium, magnesium, sodium, potassium or titanium. Preferably the metal is lithium. 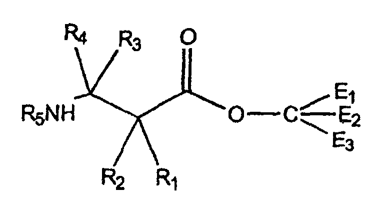 comprising reacting a β-lactam with a metal alkoxide, the β-lactam having the formula 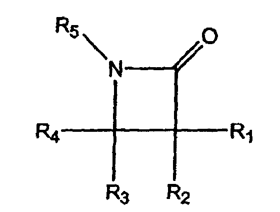 and the metal alkoxide having the formula

MOCE₁E₂E₃

wherein
R₁ is -OR₆, -SR₇, or -NR₈R₉;
R₂ is hydrogen, alkyl, alkenyl, alkynyl, aryl, or heteroaryl;
R₃ and R₄ are independently hydrogen, alkyl, alkenyl, alkynyl, aryl, heteroaryl, or acyl, provided, however, that R₃ and R₄ are not both acyl;
R₅ is -COR₁₀, -COOR₁₀, -COSR₁₀, -CONR₈R₁₀, -SO₂R₁₁, or -POR₁₂R₁₃,
R₆ is hydrogen, alkyl, alkenyl, alkynyl, aryl, heteroaryl, hydroxy protecting group, or a functional group which increases the water solubility of the taxane derivative,
R₇ is alkyl, alkenyl, alkynyl, aryl, heteroaryl, or sulfhydryl protecting group,
R₈ is hydrogen, alkyl, alkenyl, alkynyl, aryl, or heteroaryl;
R₉ is an amino protecting group;
R₁₀ is alkyl, alkenyl, alkynyl, aryl, or heteroaryl,
R₁₁ is alkyl, alkenyl, alkynyl, aryl, heteroaryl, - OR₁₀, or -NR₈R₁₄,
R₁₂ and R₁₃ are independently alkyl, alkenyl, alkynyl, aryl, heteroaryl, -OR₁₀, or -NR₈R₁₄,
R₁₄ is hydrogen, alkyl, alkenyl, alkynyl, aryl, or heteroaryl;
E₁, E₂ and E₃ are independently hydrogen, hydrocarbon or cyclic, provided, at least one of E₁, E₂ and E₃ is other than hydrogen. Preferably, in such a mouss two of E₁, E₂, and E₃ together with the carbon to which they are attached comprise a mono- or polycyclic skeleton.

The metal alkoxide and β-lactam may be selected so as to provide a process for preparing taxol, taxotere and other biologically active taxane derivatives having the following structural formula: 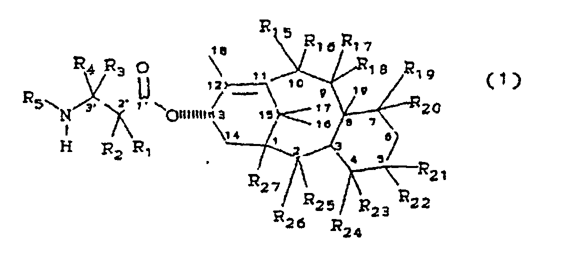 wherein
R₁ - R₁₄ are as previously defined,
R₁₅ and R₁₆ are independently hydrogen, hydroxy, lower alkanoyloxy, alkenoyloxy, alkynoyloxy, aryloyloxy or R₁₅ and R₁₆ together form an oxo;
R₁₇ and R₁₈ are independently hydrogen or lower alkanoyloxy, alkenoyloxy, alkynoyloxy, or aryloyloxy or R₁₇ and R₁₈ together form an oxo;
R₁₉ and R₂₀ are independently hydrogen or hydroxy or lower alkanoyloxy, alkenoyloxy, alkynoyloxy, or aryloyloxy;
R₂₁ and R₂₂ are independently hydrogen or lower alkanoyloxy, alkenoyloxy, alkynoyloxy, or aryloyloxy or R₂₁ and R₂₂ together form an oxo;
R₂₃ and R₂₄ are independently hydrogen or hydroxy or lower alkanoyloxy, alkenoyloxy, alkynoyloxy, or aryloyloxy; or
R₂₃ and R₂₄ together form an oxo or methylene or
R₂₃ and R₂₄ together with the carbon atom to which they are attached form an oxirane ring or
R₂₃ and R₂₂ together with the carbon atom to which they are attached form an oxetane ring;
R₂₅ is hydrogen, hydroxy, or lower alkanoyloxy, alkenoyloxy, alkynoyloxy, or aryloyloxy or ;
R₂₆ is hydrogen, hydroxy, or lower alkanoyloxy, alkenoyloxy, alkynoyloxy, or aryloyloxy; or R₂₆ and R₂₅ taken together form an oxo; and
R₂₇ is hydrogen, hydroxy or lower alkoxy, alkanoyloxy, alkenoyloxy, alkynoyloxy, or aryloyloxy.

Briefly, therefore, the taxane derivatives are prepared by reacting a β-lactam (2) with a metal alkoxide having the bi-, tri- or tetracyclic taxane nucleus to form a β-amido ester intermediate. The intermediate is then converted to the taxane derivative. β-lactam (2) has the general formula: 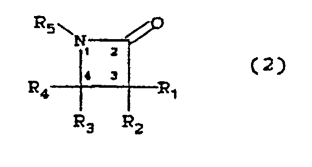 wherein R₁ - R₅ are as previously defined. The metal alkoxide preferably has the tricyclic taxane nucleus corresponding to the general formula: 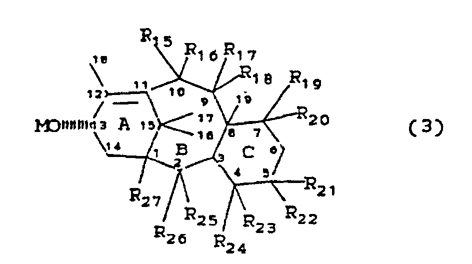 wherein M is a metal, and R₁₅-R₂₇ are as previously defined. Most preferably, the metal alkoxide has the tetracyclic taxane nucleus corresponding to metal alkoxide (3) wherein R₂₂ and R₂₃ together form an oxetane ring.

Other objects and features of this invention will be in part apparent and in part pointed out hereinafter.

### DETAILED DESCRIPTION

A process is described herein for preparing substituted isoserine esters, in general, and taxol, taxotere and other taxane derivatives which are biologically active using β-lactam (2), the structure of which is depicted hereinbelow: 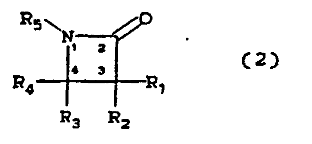 wherein R₁, R₂, R₃, R₄ and R₅ are as previously defined.

R₅ of β-lactam (2) is preferably -COR₁₀ with R₁₀ being aryl, heteroaryl, p-substituted phenyl, or lower alkoxy, and most preferably phenyl, methoxy, ethoxy, tert-butoxy ("tBuO"; (CH₃)₃CO-), or 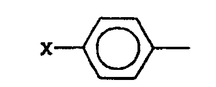 wherein X is Cl, Br, F, CH₃O-, or NO₂-. Preferably R₂ and R₄ are hydrogen or lower alkyl. R₃ is preferably aryl, most preferably, naphthyl, phenyl, 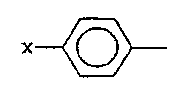 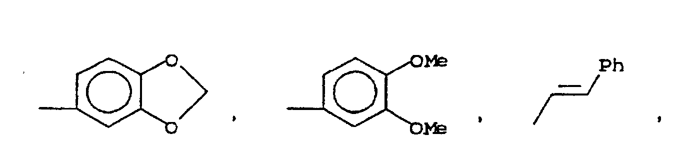 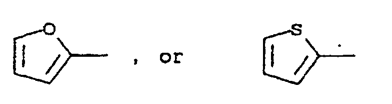 wherein X is as previously defined, Me is methyl and Ph is phenyl. Preferably, R₁ is selected from -OR₆, -SR₇ or - -NR₈R₉ wherein R₆, R₇ and R₉, are hydroxy, sulfhydryl, and amine protecting groups, respectively, and R₈ is hydrogen, alkyl, alkenyl, alkynyl, aryl, or heteroaryl. Most preferably, R₁ is -OR₆ wherein R₆ is triethylsilyl ("TES"), 1-ethoxyethyl ("EE") or 2,2,2-trichloroethoxymethyl.

The β-lactam alkyl groups, either alone or with the various substituents defined hereinabove are preferably lower alkyl containing from one to six carbon atoms in the principal chain and up to 15 carbon atoms. They may be straight or branched chain and include methyl, ethyl, propyl, isopropyl, butyl, isobutyl, tert-butyl, amyl, hexyl, and the like.

The β-lactam alkenyl groups, either alone or with the various substituents defined hereinabove are preferably lower alkenyl containing from two to six carbon atoms in the principal chain and up to 15 carbon atoms. They may be straight or branched chain and include ethenyl, propenyl, isopropenyl, butenyl, isobutenyl, pentenyl, hexenyl, and the like.

The β-lactam alkynyl groups, either alone or with the various substituents defined hereinabove are preferably lower alkynyl containing from two to six carbon atoms in the principal chain and up to 15 carbon atoms. They may be straight or branched chain and include ethynyl, propynyl, butynyl, isobutynyl, pentynyl, hexynyl, and the like.

The β-lactam aryl moieties described, either alone or with various substituents, contain from 6 to 15 carbon atoms and include phenyl, α-naphthyl or β-naphthyl, etc. Substituents include alkanoxy, protected hydroxy, halogen, alkyl, aryl, alkenyl, acyl, acyloxy, nitro, amino, amido, etc. Phenyl is the more preferred aryl.

As noted above, R₁ of β-lactam (2) may be -OR₆ with R₆ being alkyl, acyl, ethoxyethyl ("EE"), triethylsilyl ("TES"), 2,2,2-trichloroethoxymethyl, or other hydroxyl protecting group such as acetals and ethers, i.e., methoxymethyl ("MOM"), benzyloxymethyl; esters, such as acetates; carbonates, such as methyl carbonates; and alkyl and aryl silyl such as triethylsilyl, trimethylsilyl, dimethyl-t-butylsilyl, dimethylarylsilyl, dimethyl-heteroarylsilyl, and triisopropylsilyl, and the like. A variety of protecting groups for the hydroxyl group and the synthesis thereof may be found in "Protective Groups in Organic Synthesis" by T. W. Greene, John Wiley and Sons, 1981. The hydroxyl protecting group selected should be easily removed under conditions that are sufficiently mild, e.g., in 48% HF, acetonitrile, pyridine, or 0.5% HCl/water/ ethanol, and/or zinc/acetic acid so as not to disturb the ester linkage or other substituents of the taxol intermediate. However, R₆ is preferably triethylsilyl, 1-ethoxyethyl or 2,2,2-trichloroethoxymethyl, and most preferably triethylsilyl.

Also as noted previously, R₇ may be a sulfhydryl protecting group and R₉ may be an amine protecting group. Sulfhydryl protecting groups include hemithioacetals such as 1-ethoxyethyl and methoxymethyl, thioesters, or thiocarbonates. Amine protecting groups include carbamates, for example, 2,2,2-trichloroethylcarbamate or tertbutylcarbamate. A variety of sulfhydryl and amine protecting groups may be found in the above-identified text by T. W. Greene.

Since β-lactam (2) has several asymmetric carbons, it is known to those skilled in the art that the compounds of the present invention having asymmetric carbon atoms may exist in diastereomeric, racemic, or optically active forms. All of these forms are contemplated within the scope of this invention. More specifically, the present invention includes enantiomers, diastereomers, racemic mixtures, and other mixtures thereof.

β-lactam (2) can be prepared from readily available materials, as is illustrated in schemes A and B below: 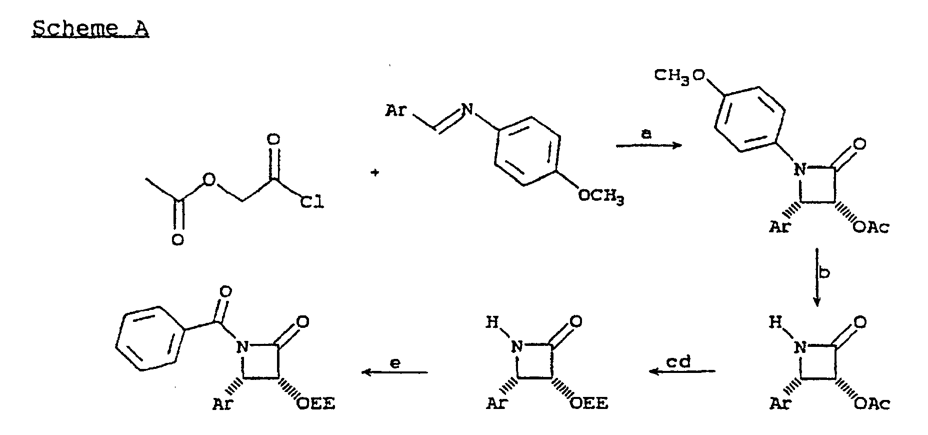 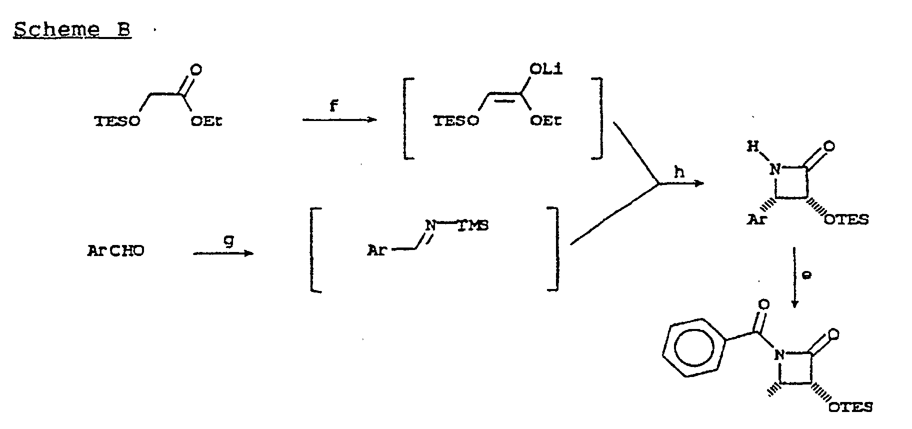 reagents: (a) triethylamine, CH₂Cl₂, 25°C, 18h; (b) 4 equiv ceric ammonium nitrate, CH₃CN, -10°C, 10 min; (c) KOH, THF, H₂O, O°C, 30 min; (d) ethyl vinyl ether, THF, toluene sulfonic acid (cat.), O°C, 1.5h; (e) n-butyllithium, ether, -78°C, 10 min; benzoyl chloride, -78°C, 1h; (f) lithium diisopropyl amide, THF -78°C to -50°C; (g) lithium hexamethyldisilazide, THF -78°C to 0°C; (h) THF, -78°C to 25°C, 12h.

The starting materials are readily available. In scheme A, α-acetoxy acetyl chloride is prepared from glycolic acid, and, in the presence of a tertiary amine, it cyclocondenses with imines prepared from aldehydes and p-methoxyaniline to give 1-p-methoxyphenyl-3-acyloxy-4-arylazetidin-2-ones. The p-methoxyphenyl group can be readily removed through oxidation with ceric ammonium nitrate, and the acyloxy group can be hydrolyzed under standard conditions familiar to those experienced in the art to provide 3-hydroxy-4-arylazetidin-2-ones. The 3-hydroxyl group is protected with 1-ethoxyethyl, but may be protected with variety of standard protecting groups such as the triethylsilyl group or other trialkyl (or aryl) silyl groups. In Scheme B, ethyl-α-triethylsilyloxyacetate is readily prepared from glycolic acid.

The racemic β-lactams may be resolved into the pure enantiomers prior to protection by recrystallization of the corresponding 2-methoxy-2-(trifluoromethyl) phenylacetic esters. However, the reaction described hereinbelow in which the ß-amido ester side chain is attached has the advantage of being highly diastereo-selective, thus permitting the use of a racemic mixture of side chain precursor.

The 3-(1-ethoxyethoxy)-4-phenylazetidin-2-one of scheme A and the 3-(1-triethylsilyloxy)-4-phenylazetidin-2-one of scheme B can be converted to ß-lactam (2), by treatment with a base, preferably n-butyllithium, and an acyl chloride, alkylchloroformate, sulfonyl chloride, phosphinyl chloride or phosphoryl chloride at -78 °C or below.

The process described herein is particularly useful for the esterification of mono- or polycyclic metal alkoxides which are represented by the formula 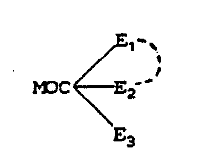 in which E₁, E₂ and the carbon to which they are attached define a carbocyclic and/or heterocyclic skeleton which may be mono- or polycyclic and E₃ is hydrogen or hydrocarbon, preferably lower alkyl. Most preferably, the carbocyclic and/or heterocyclic skeleton comprises about 6 to 20 atoms and the hetero atoms are oxygen. The cyclic skeleton may be hydrocarbon and/or heterosubstituted with heterosubstituents including, for example, esters, ethers, amines, alcohols, protected alcohols, carbonyl groups, halogens, oxygen, substituted oxygen or substituted nitrogen.

When the metal alkoxides have the bi-, tri- or tetracyclic taxane nucleus, the process described herein may advantageously be used to prepare taxane derivatives, many of which have been found to have significant biological activity. As used herein, a metal alkoxide having the bicyclic taxane nucleus has the carbocyclic skeleton corresponding to rings A and B of metal alkoxide (3): 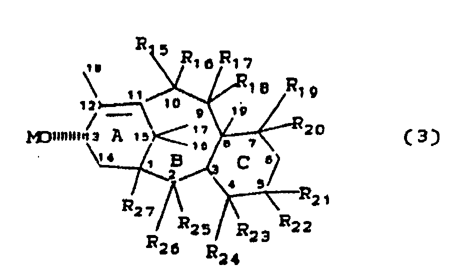 M and R₁₅-R₂₇ are as previously defined. A metal alkoxide having the tricyclic taxane nucleus has the carbocyclic skeleton corresponding to rings A, B and C of metal alkoxide (3). A metal alkoxide having the tetracyclic taxane nucleus has carbocyclic rings A, B and C of metal alkoxide (3) and the oxetane ring defined by R₂₂, R₂₃, and the carbons to which they are attached.

Preferably, the metal alkoxide used in the process described herein is metal alkoxide (3). Most preferably, R₁₅ is -OT₂ or -OCOCH₃; R₁₆ is hydrogen; R₁₇ and R₁₈ together form an oxo; R₁₉ is -OT₁; R₂₀ and R₂₁ are hydrogen; R₂₂ and R₂₃ together with the carbons to which they are attached form an oxetane ring; R₂₄ is CH₃COO-; R₂₅ is PhCOO-; R₂₆ is hydrogen; R₂₇ is hydroxy; and T₁ and T₂ are independently hydrogen or hydroxy protecting groups.

Metal substituent, M, of metal alkoxide (3) is a Group IA, IIA, IIIA, lanthanide or actinide element or a transition, Group IIIA, IVA, VA or VIA metal. Preferably, it is a Group IA, IIA or transition metal, and most preferably, it is lithium, magnesium, sodium, potassium or titanium.

The metal alkoxide alkyl groups, either alone or with the various substituents defined hereinabove are preferably lower alkyl containing from one to six carbon atoms in the principal chain and up to 10 carbon atoms. They may be straight or branched chain and include methyl, ethyl, propyl, isopropyl, butyl, isobutyl, tert-butyl, hexyl, and the like.

The metal alkoxide alkenyl groups, either alone or with the various substituents defined hereinabove are preferably lower alkenyl containing from two to six carbon atoms in the principal chain and up to 10 carbon atoms. They may be straight or branched chain and include ethenyl, propenyl, isopropenyl, butenyl, isobutenyl, hexenyl, and the like.

The metal alkoxide alkynyl groups, either alone or with the various substituents defined hereinabove are preferably lower alkynyl containing from two to six carbon atoms in the principal chain and up to 10 carbon atoms. They may be straight or branched chain and include ethynyl, propynyl, butynyl, isobutynyl, hexynyl, and the like.

Exemplary alkanoyloxy include acetate, propionate, butyrate, valerate, isobutyrate and the like. The more preferred alkanoyloxy is acetate.

The metal alkoxide aryl moieties, either alone or with various substituents contain from 6 to 10 carbon atoms and include phenyl, α-naphthyl or β-naphthyl, etc. Substituents include alkanoxy, hydroxy, halogen, alkyl, aryl, alkenyl, acyl, acyloxy, nitro, amino, amido, etc. Phenyl is the more preferred aryl.

Metal alkoxides (3) are prepared by reacting an alcohol having two to four rings of the taxane nucleus and a C-13 hydroxyl group with an organometallic compound in a suitable solvent. Preferably, the alcohol is a derivative of baccatin III or 10-deacetyl baccatin III having the structure 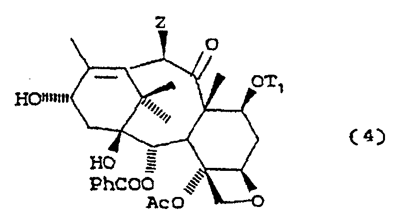 wherein T₁ is a hydroxy protecting group, and Z is -OT₂ wherein T₂ is acyl, preferably acetyl, or other hydroxy protecting group. Most preferably, the alcohol is a protected baccatin III, in particular, 7-0-triethylsilyl baccatin III (which can be obtained as described by Greene, et al. in JACS 110, 5917 (1988) or by other routes) or 7,10-bis-O-triethylsilyl baccatin III.

As reported in Greene et al., 10-deacetyl baccatin III is converted to 7-O-triethylsilyl-10-deacetyl baccatin III according to the following reaction scheme: 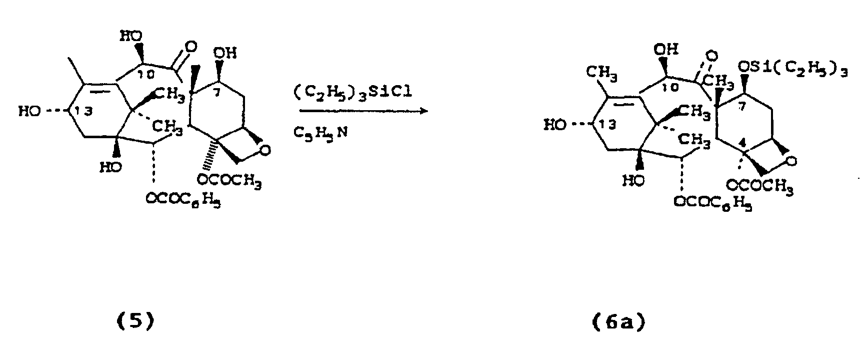 Under what is reported to be carefully optimized conditions, 10-deacetyl baccatin III is reacted with 20 equivalents of (C₂H₅)₃SiCl at 23°C under an argon atmosphere for 20 hours in the presence of 50 ml of pyridine/mmol of 10-deacetyl baccatin III to provide 7-triethylsilyl-10-deacetyl baccatin III (6a) as a reaction product in 84-86% yield after purification. The reaction product is then acetylated with 5 equivalents of CH₃COCl and 25 mL of pyridine/mmol of (6a) at 0 °C under an argon atmosphere for 48 hours to provide 86% yield of 7-O-triethylsilyl baccatin III (6b). Greene, et al. in JACS 110, 5917 at 5918 (1988).

Alternatively, 7-triethylsilyl-10-deacetyl baccatin III (6a) can be protected at C-10 oxygen with an acid labile hydroxyl protecting group. For example, treatment of (6a) with n-butyllithium in THF followed by triethylsilyl chloride (1.1 mol equiv.) at 0°C gives 7,10-bis-O-triethylsilyl baccatin III (6c) in 95% yield. Also, (6a) can be converted to 7-O-triethylsilyl-10-(1-ethoxyethyl) baccatin III (6d) in 90% yield by treatment with excess ethyl vinyl ether and a catalytic amount of methane sulfonic acid. These preparations are illustrated in the reaction scheme below.

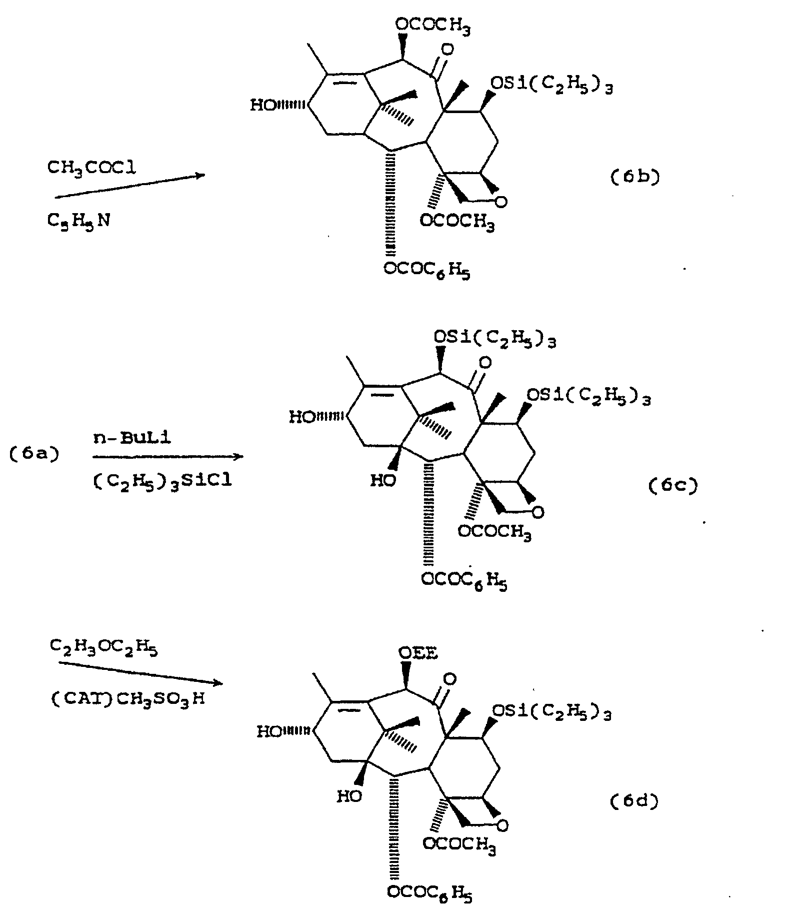

7-O-triethylsilyl baccatin III (6b), 7,10-bis-O-triethylsilyl baccatin III (6c), or 7-0-triethylsilyl-10-(1-ethoxyethyl) baccatin III (6d) is reacted with an organometallic compound such as n-butyllithium in a solvent such as tetrahydrofuran (THF), to form the metal alkoxide 13-O-lithium-7-O-triethylsilyl baccatin III (7b) 13-O-lithium-7,10-bis-O-triethylsilyl baccatin III (7c), or 13-O-lithium-7-O-triethylsilyl-10-(1-ethoxyethyl) baccatin III (7d) as shown in the following reaction scheme:

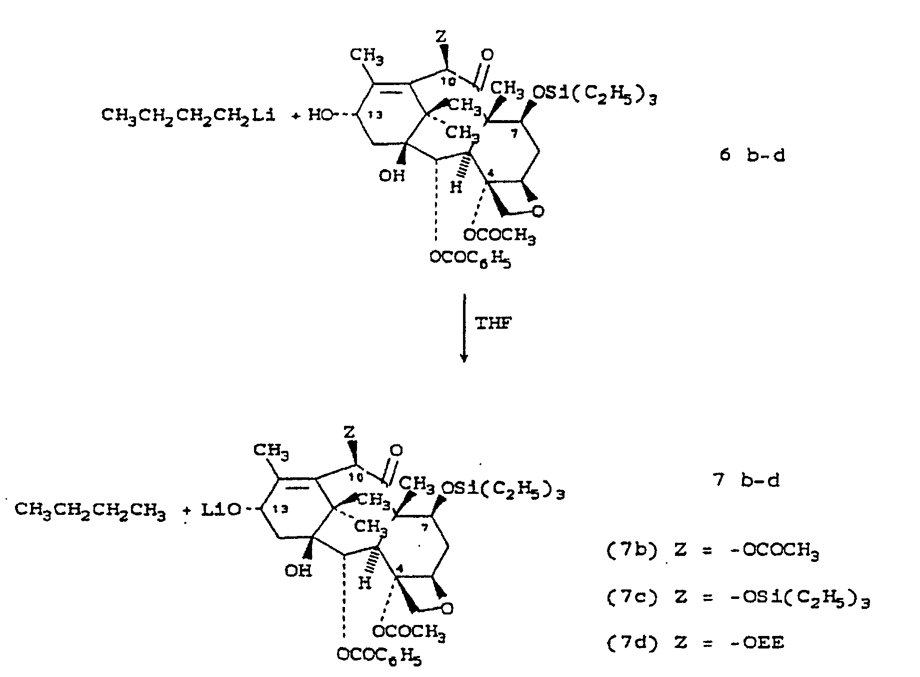

As illustrated in the following reaction scheme, a suitable metal alkoxide of the present invention such as 13-O-lithium-7-O-triethylsilyl baccatin III derivative (7b, 7c, or 7d) reacts with a β-lactam of the present invention to provide an intermediate (8b, 8c, or 8d) in which the C-7 hydroxyl group is protected with a triethylsilyl or 1-ethoxyethyl group.

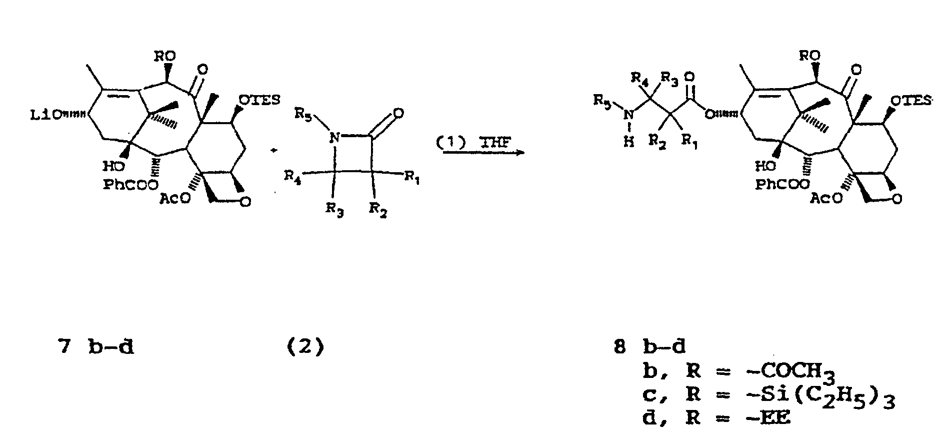

Intermediate compound (8b) readily converts to taxol when R₁ is -OR₆, R₂ and R₃ are hydrogen, R₄ is phenyl, R₅ is benzoyl and R₆ is a hydroxy protecting group such as triethylsilyl. Intermediate compound (8c) readily converts to taxotere when R₁ is -OR₆, R₂ and R₃ are hydrogen, R₄ is phenyl, R₅ is tertbutoxycarbonyl and R₆ is a hydroxy protecting group such as triethylsilyl. Intermediate compound (8d) readily converts to 10-deacetyl taxol when R₁ is -OR₆, R₂ and R₃ are hydrogen, R₄ is phenyl, R₅ is benzoyl, and R₆ is a hydroxy protecting group such as triethylsilyl. Intermediate compounds (8b, 8c and 8d) may be converted to the indicated compounds by hydrolyzing the triethylsilyl and 1-ethoxyethyl groups under mild conditions so as not to disturb the ester linkage or the taxane derivative substituents.

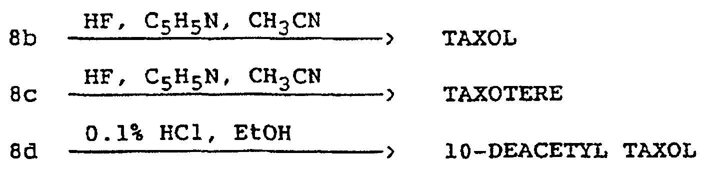

Other taxane derivatives may readily be prepared by selection of the proper substituents R₁ - R₅ of β-lactam (2) or R₁₅ - R₂₇ of metal alkoxide (3). The preparation of such other compounds is illustrated in the examples which follow.

Both the conversion of the alcohol to the metal alkoxide and the ultimate synthesis of the taxol can take place in the same reaction vessel. Preferably, the β-lactam is added to the reaction vessel after formation therein of the metal alkoxide.

The organometallic compound n-butyllithium is preferably used to convert the alcohol to the corresponding metal alkoxide, but other sources of metallic substituent such as lithium diisopropyl amide, other lithium or magnesium amides, ethylmagnesium bromide, methylmagnesium bromide, other organolithium compounds, other organomagnesium compounds, organosodium, organotitanium, organozirconium, organozinc, organocadmium or, organopotassium or the corresponding amides may also be used. Organometallic compounds are readily available, or may be prepared by available methods including reduction of organic halides with metal. Lower alkyl halides are preferred. For example, butyl bromide can be reacted with lithium metal in diethyl ether to give a solution of n-butyllithium in the following manner:

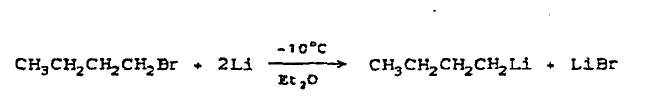

Alternatively, the lithium alkoxide may be induced to undergo exchange with metal halides to form alkoxides of aluminum, boron, cerium, calcium, zirconium or zinc.

Although THF is the preferred solvent for the reaction mixture, other ethereal solvents, such as dimethoxyethane, or aromatic solvents may also be suitable. Certain solvents, including some halogenated solvents and some straight-chain hydrocarbons in which the reactants are too poorly soluble, are not suitable. Other solvents are not appropriate for other reasons. For example, esters are not appropriate for use with certain organometallic compounds such as n-butyllithium due to incompatibility therewith.

Although the reaction scheme disclosed herein is directed to the synthesis of certain taxol derivatives, it can be used with modifications in either the β-lactam or the tetracyclic metal alkoxide. Therefore metal alkoxides other than 13-O-lithium-7-O-triethylsilyl baccatin III may be used to form a taxol intermediate according to the method of this invention. The β-lactam and the tetracyclic metal alkoxide can be derived from natural or unnatural sources, to prepare other synthetic taxols, taxol derivatives, 10-deacetyltaxols, and the enantiomers and diastereomers thereof contemplated within the present invention.

The process of the invention also has the important advantage of being highly diastereoselective. Therefore racemic mixtures of the side chain precursors may be used. Substantial cost savings may be realized because there is no need to resolve racemic β-lactams into their pure enantiomers. Additional cost savings may be realized because less side chain precursor, e.g., 60-70% less, is required relative to prior processes.

The water solubility of compounds of formula (1) may be improved if R₁ is -OR₆ and R₁₉ is -OT₁, and R₆ and/or T₁ are a functional group which increases solubility, such as -COGCOR¹ wherein:
G is ethylene, propylene, CHCH, 1,2-cyclohexylene, or 1,2-phenylene;
R¹ = OH base, NR²R³, OR³, SR³, OCH₂CONR⁴R⁵, or OH;
R² = hydrogen or methyl;
R³ = (CH₂)ₙNR⁶R⁷ or (CH₂)ₙ N^{⊕}R⁶R⁷R⁸X₁^{⊖};
n = 1 to 3;
R⁴ = hydrogen or lower alkyl containing 1 to 4 carbons;
R⁵ = hydrogen, lower alkyl containing 1 to 4 carbons, benzyl, hydroxyethyl, CH₂CO₂H, or dimethylaminoethyl
R⁶ and R⁷= lower alkyl containing 1 or 2 carbons, or benzyl;
or R⁶ and R⁷ together with the nitrogen atom of NR⁶R⁷ forms one of the following rings 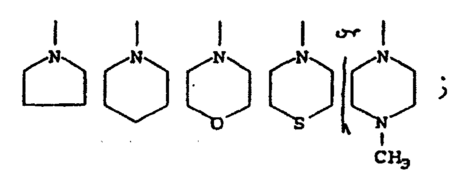
R⁸ = lower alkyl containing 1 or 2 carbons or benzyl;
X₁^{⊖} = halide; and
base = NH₃, (HOC₂H₄)₃N, N(CH₃)₃, CH₃N(C₂H₄OH)₂, NH₂(CH₂)₆NH₂, N-methylglucamine, NaOH₂ or KOH.
The preparation of compounds in which R₆ or T₁ is -COGCOR¹ is set forth in Hangwitz U.S. Patent 4,942,184.

The following examples illustrate the invention.

### EXAMPLE 1

### Preparation of 2'-ethoxyethyl-7-triethylsilyl taxol, and subsequently taxol, from racemic β-lactam:

To a solution of 7-triethylsilyl baccatin III (20mg, 0.028 mmol) in 1 ml of THF at -78°C was added dropwise 0.17 ml of a 0.164M solution of nBuLi in hexane. After 30 min at -78°C, a solution of cis-1-benzoyl-3-(1-ethoxyethoxy)-4-phenylazetidin-2-one (47.5 mg, 0.14 mmol) in 1 ml of THF was added dropwise to the mixture. The solution was allowed to slowly warm (over 1.5 h) to 0°C and was then stirred at 0°C for 1 h and 1 ml of a 10% solution of AcOH in THF was added. The mixture was partitioned between saturated aqueous NaHCO₃ and 60/40 ethyl acetate/hexane. Evaporation of the organic layer gave a residue which was purified by flash chromatography to give 23 mg (80%) of (2'R, 3'S)-2'-ethoxyethyl-7-tri-ethylsilyl taxol and 3.5 mg (13%) of 2',3'-epi(2'S, 3'R)-2'-ethoxyethyl-7-triethylsilyl taxol.

A 5 mg sample of (2'R, 3'S)-2'-ethoxyethyl-7-triethylsilyl taxol was dissolved in 2 ml of ethanol, and 0.5 ml of 0.5% aqueous HCl solution was added. The mixture was stirred at 0°C for 30 h and. diluted with 50 ml of ethyl acetate. The solution was extracted with 20 ml of saturated aqueous sodium bicarbonate solution, dried over sodium sulfate and concentrated. The residue was purified by flash chromatography to provide 4.5 mg (ca.90%) taxol, which was identical with an authentic sample in all respects.

A 5 mg sample of 2',3'-epi(2'S,3'R)-2'-ethoxy-ethyl-7-triethylsilyl taxol was dissolved in 2 ml of ethanol and 0.5 ml of 0.5% aqueous HCl solution was added. The mixture was stirred at 0°C for 30 h and diluted with 50 ml of ethyl acetate. The solution was extracted with 20 ml of saturated aqueous sodium bicarbonate solution, dried over sodium sulfate and concentrated. The residue was purified by flash chromatography to provide 4.5 mg (ca.90%) of 2',3'-epitaxol.

### EXAMPLE 2

### Preparation of 2',7-(bis)triethylsilyl taxol, and subsequently taxol, from racemic β-lactam:

To a solution of 7-triethylsilyl baccatin III (100mg, 0.143 mmol) in 1 ml of THF at -45°C was added dropwise 0.087 ml of a 1.63M solution of nBuLi in hexane. After 1 h at -45°C, a solution of cis-1-benzoyl-3-triethylsilyloxy)-4-phenylazetidin-2-one (274 mg, 0.715 mmol) in 1 ml of THF was added dropwise to the mixture. The solution was allowed to warm to 0°C and held at 0°C for 1 h. One ml of a 10% solution of AcOH in THF was added. The mixture was partitioned between saturated aqueous NaHCO₃ and 60/40 ethyl acetate/hexane. Evaporation of the organic layer gave a residue which was purified by flash chromatography followed by recrystallization to give 131 mg (85%) of (2'R, 3'S)-2',7-(bis)triethylsilyl taxol and 15 mg (10%) of 2',3'-epi(2'S,3'R)-2',7-(bis)triethylsilyl taxol.

To a solution of 121.3 mg (0.112 mmol) of (2'R, 3'S)-2',7-(bis)triethylsilyl taxol in 6 ml of acetonitrile and 0.3 ml of pyridine at 0°C was added 0.9 ml of 48% aqueous HF. The mixture was stirred at 0°C for 8 h, then at 25°C for 6 h. The mixture was partitioned between saturated aqueous sodium bicarbonate and ethyl acetate. Evaporation of the ethyl acetate solution gave 113 mg of material which was purified by flash chromatography and recrystallization to give 94 mg (98%) taxol, which was identical with an authentic sample in all respects.

To a solution of 5 mg of (2'R, 3'S)-2',7-(bis) triethylsilyl taxol in 0.5 ml of acetonitrile and 0.03 ml of pyridine at 0°C was added 0.09 ml of 48% aqueous HF. The mixture was stirred at 0°C for 8 h, then at 25°C for 6 h. The mixture was partitioned between saturated aqueous sodium bicarbonate and ethyl acetate. Evaporation of the ethyl acetate solution gave 5 mg of material which was purified by flash chromatography and recrystallization to give 4.6 mg (ca. 95%) of 2',3'-epitaxol.

### EXAMPLE 3

### Preparation of 2',7-(bis)triethylsilyl taxol, and subsequently taxol, from optically active β-lactam:

To a solution of 7-triethylsilyl baccatin III (100 mg, 0.143 mmol) in 1 ml of THF at -45°C was added dropwise 0.087 ml of a 1.63M solution of nBuLi in hexane. After 1 h at -45°C, a solution of (+)-cis-1-benzoyl-3-triethylsilyloxy-4-phenylazetidin-2-one (82 mg, 0.215 mmol) in 1 ml of THF was added dropwise to the mixture. The solution was allowed to warm to 0°C and held at 0°C for 2 hours. One ml of a 10% solution of AcOH in THF was added. The mixture was partitioned between saturated aqueous NaHCO₃ and 60/40 ethyl acetate/hexane. Evaporation of the organic layer gave a residue which was purified by flash chromatography followed by recrystallization to give 145 mg (94%) of (2'R, 3'S)-2',7-(bis)triethylsilyl taxol.

To a solution of 121.3 mg (0.112 mmol) of (2'R, 3'S)-2',7-(bis)triethylsilyl taxol in 6 ml of acetonitrile and 0.3 ml of pyridine at 0°C was added 0.9. ml of 48% aqueous HF. The mixture was stirred at 0°C for 8 h, then at 25°C for 6 h. The mixture was partitioned between saturated aqueous sodium bicarbonate and ethyl acetate. Evaporation of the ethyl acetate solution gave 113 mg of material which was purified by flash chromatography and recrystallization to give 94 mg (98%) taxol, which was identical with an authentic sample in all respects.

### EXAMPLE 4

### Preparation of taxotere.

To a solution of 7,10-bis-triethylsilyl baccatin III (200 mg, 0.248 mmol) in 2 mL of THF at -45 °C was added dropwise -0.174 mL of a 1.63M solution of nBuLi in hexane. After 0.5 h at -45 °C, a solution of cis-1-(tert-butoxycarbonyl)-3-triethylsilyloxy-4-phenylazetidin-2-one (467 mg, 1.24 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 280 mg of crude 2',7,10-tris-triethylsilyl taxotere.

To a solution of 280 mg of the crude product obtained from the previous reaction in 12 mL of acetonitrile and 0.6 mL of pyridine at 0 °C was added 1.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 215 mg of material which was purified by flash chromatography to give 190 mg (95%) of taxotere, which was recrystallized from methanol/water. All analytical and spectral data were identical with that reported for taxotere in U.S. Patent 4,814,470.

### EXAMPLE 5

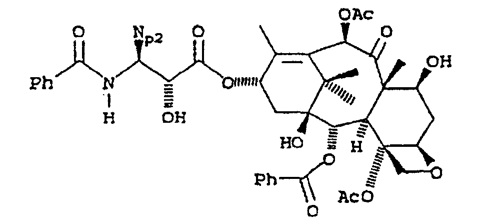

wherein Nₚ₂ is

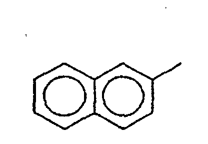

### Preparation of 3'-desphenyl-3'-(2-naphthyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(2-naphthyl)azetidin-2-one (620 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl-3'-desphenyl-3'-(2-naphthyl) taxol and a small amount of the (2'S,3'R) isomer.

To a solution of 320 mg (0.283 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 255 mg of material which was purified by flash chromatography to give 166 mg (64%) of 3'-desphenyl-3'-(2-naphthyl) taxol, which was recrystallized from methanol/water.
m.p 164-165 °C; [α]²⁵_{Na} -52.6° (c 0.005, CHCl₃).
¹H NMR (CDCl₃, 300 MHz) δ 8.14 (d, J = 7.3 Hz, 2H, benzoate ortho), 7.96 (m, 1H, aromatic), 7.90 (m, 1H, aromatic), 7.85 (m, 2H, aromatic), 7.76 (m, 2H, aromatic), 7.60 (m, 3H, aromatic), 7.52 (m, 4H, aromatic) , 7.41 (m, 2H, aromatic), 7.01 (d, J = 8.8 Hz, 1H, NH), 6.27 (s, 1H, H10), 6.26 (dd, J = 9.2, 9.2 Hz, 1H, H13), 5.97 (dd, J = 8.8, 2.5 Hz, 1H, H3'), 5.68 (d, J = 7.1 Hz, 1H, H2β), 4 .93 (m, 1H, H5), 4.92 (m, 1H, H2'), 4.39 (m, 1H, H7), 4.30 (d, J = 8.5 Hz, 1H, H20α), 4.20 (d, J = 8.5 Hz, 1H, H20β), 3.81 (d, J = 7.1 Hz, 1H, H3), 3.60 (d, J m 5 Hz, 1H, 2'OH), 2.48 (m, 1H, H6α), 2 .45 (br, 1H, 7OH), 2 .39 (s, 3H, 4Ac), 2 .30 (m, 2H, H14), 2.24 (s, 3H, 10Ac), 1.83 (m, 1H, H6β), 1.82 (br s, 3H, Me18), 1.68 (s, 1H, 10H), 1.68 (s, 3H, Me19), 1.24 (s, 3H, Me17), 1.14 (s, 3H, Me16).

### EXAMPLE 6

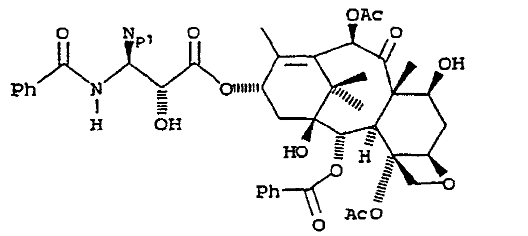

wherein Nₚₗ is

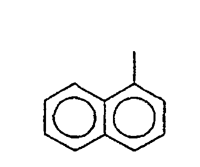

### Preparation of 3'-desphenyl-3'-(1-naphthyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(1-naphthyl)azetidin-2-one (620 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 325 mg of a mixture containing (2'R,3'S)-2',7-(bis)triethylsilyl-3'-desphenyl-3'-(1-naphthyl) taxol and a small amount of the (2'S,3'R) isomer.

To a solution of 325 mg (0.287 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 260 mg of material which was purified by flash chromatography to give 166 mg (64%) of 3'-(1-naphthyl) taxol, which was recrystallized from methanol/water.
m.p. 164-165 °C;[α]²⁵_{Na}-52.6° (c 0.005, CHCl₃).
¹H NMR (CDCl₃, 300 MHz) δ 8.11 (d, J = 7.1 Hz, 2H, benzoate ortho), 8.11 (m, 3H, aromatic), 7.91 (m, 3H, aromatic), 7.70 (m, 2H, aromatic), 7.63-7.46 (m, 7H, aromatic), 6.75 (d, J = 8. 8 Hz, 1H, NH) , 6.52 (dd, J = 8.8, 1.6 Hz, 1H, H3'), 6.27 (s, 1H, H10), 6.27 (dd, J = 9.1, 9.1 Hz, 1H, H13), 5.68 (d, J = 7.1 Hz, 1H, H2β), 4.85 (dd, J = 7.6, 2.2 Hz, 1H, H5), 4.97 (dd, J - 1.6 Hz, 1H, H2'), 4.39 (m, 1H, H7), 4.24 (d, J = 8.5 Hz, 1H, H20α), 4.17 (d, J = 8.5 Hz, 1H, H20β), 3.80 (d, J = 7.1 Hz, 1H, H3), 3.65 (br, 1H, 2'OH), 2.55 (m, 1H, H6α), 2.48 (br, 1H, 7OH), 2.41 (s, 3H, 4Ac), 2.38 (m, 1H, H14), 1.96 (s, 3H, 10Ac), 1.86 (m, 1H, H6β), 1.80 (br s, 3H, Me18), 1.76 (s, 1H, 1OH), 1.69 (s, 3H, Me19), 1.28 (s, 3H, Me17), 1.16 (s, 3H, Me16).

### EXAMPLE 7

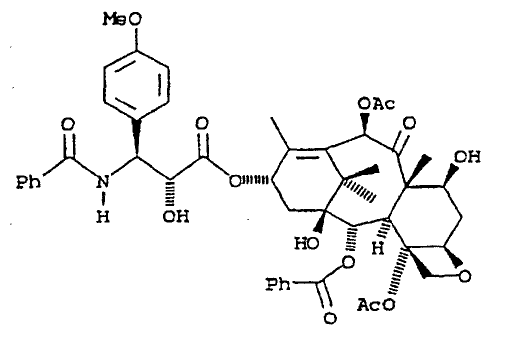

### Preparation of 3'-desphenyl-3'-(4-methoxyphenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-9-(4-methoxyphenyl)azetidin-2-one (590 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl-3'-desphenyl-3'-(4-methoxyphenyl) taxol and a small amount of the (2'S,3'R) isomer.

To a solution of 320 mg (0.288 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 255 mg of material which was purified by flash chromatography to give 172 mg (68%) of 3'-desphenyl-3'-(4-methoxyphenyl) taxol, which was recrystallized from methanol/water.
m.p. 174-176 °C;[α]²⁵_{Na}-48.86° (c 0.05, CHCl₃).
¹H NMR (CDCl₃, 300 MHz) δ 8.12 (d, J = 7.1 Hz, 2H, benzoate ortho), 7.72 (m, 2H, aromatic), 7.59 (m, 1H, aromatic), 7.53-7.36 (m, 8H, aromatic), 6.96 (d, J = 8.8 Hz, 1H, NH), 6.90 (m, 2H, aromatic), 6.26 (s, 1H, H10), 6.21 (dd, J = 9.3, 9.3 Hz, 1H, H13), 5.70 (dd, J = 8.8, 2.7 Hz, 1H, H3'), 5.66 (d, J = 6.8 Hz, 1H, H2ß), 4.93 (dd, J = 9.9, 2.2 Hz, 1H, H5), 4.74 (dd, J = 5 .5, 2 .7 Hz, 1H, H2'), 4.39 (m, 1H, H7), 4.29 (d, J = 8.8 Hz, 1H, H20α), 4.18 (d, J = 8.8.Hz, 1H, H20ß), 3 .78 (d, J = 6.8 Hz, 1H, H3), 3.78 (s, 3H, ArOMe), 3 . 67 (d, J = 5.5 Hz, 1H, 2'OH), 2.61 (m, 1H, H6α), 2.50 (d, J = 4.4 Hz, 1H, 7OH), 2.37 (s, 3H, 4Ac), 2.31 (m, 2H, H14), 2. 22 (s, 3H, 10Ac), 1.84 (m, 1H, H6ß), 1.79 (br s, 3H, Me18), 1.79 (s, 1H, 1OH), 1.67 (s, 3H, Me19), 1.22 (s, 3H, Me17), 1.13 (s, 3H, Me16).

### EXAMPLE 8

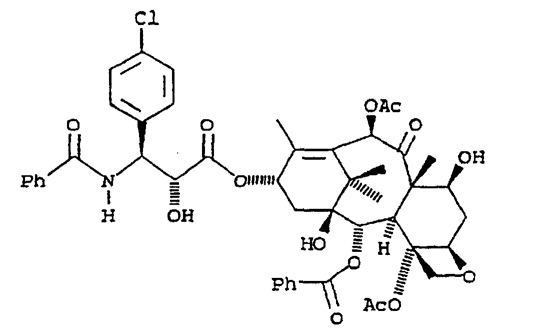

### Preparation of 3'-desphenyl-3'-(4-chlorophenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(4-chlorophenyl)azetidin-2-one (595 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl 3'-desphenyl-3'-(4-chlorophenyl) taxol and a small amount of the (2'S,3'R) isomer.

To a solution of 320 mg (0.287 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 255 mg of material which was purified by flash chromatography to give 158 mg (62%) of 3'-desphenyl-3'-(4-chlorophenyl) taxol, which was recrystallized from methanol/water.
m.p. 173-175 °C;[α]²⁵_{Na}-50.8° (c 0.01, CHCl₃).
¹H NMR (CDCl₃, 300 MHz) δ 8.13 (d, J = 7.1 Hz, 2H, benzoate ortho), 7.72 (d, J = 8.2 Hz, 2H, benzamide ortho), 7.65-7.35 (m, 10H, aromatic), 6.97 (d, J = 8.8 Hz, 1H, NH), 6.27 (s, 1H, H10), 6.25 (dd, J = 8.3, 8.3 Hz, 1H, H13), 5.78 (dd, J = 8.8, 2.2 Hz, 1H, H3' ), 5.67 (d, J - 7.1 Hz, 1H, H2β), 4 .95 (dd, J = 8.8, 2.2 Hz, 1H, H5), 4.77 (br s, 1H, H2'), 4.40 (m, 1H, H7), 4.31 (d, J = 8.2 Hz, 1H, H20α), 4.19 (d, J = 8.2 Hz, 1H, H20β), 3.80 (d, J = 7.1 Hz, 1H, H3), 3.61 (br s, 1H, 2'OH), 2.54 (m, 1H, H6α), 2.38 (s, 3H, 4Ac), 2.32 (m, 2H, H14), 2.24 (s, 3H, 10Ac), 1.85 (m, 1H, H6β), 1.80 (br s, 3H, Me18), 1.68 (s, 3H, Me19), 1.23 (s, 3H, Me17), 1.14 (s, 3H, Me16).

### EXAMPLE 9

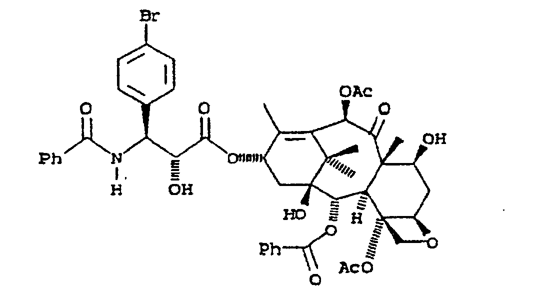

### Preparation of 3'-desphenyl-3'-(4-bromophenyl) taxol.

To a solution of 7-triethylsilyl baccatin III (200 mg, 0.286 mmol) in 2 mL of THF at -45 °C was added dropwise 0.174 mL of a 1.63M solution in nBuLi in hexane. After 0.5 h at -45 °C, a solution of cis-1-benzoyl-3-triethylsilyloxy-4-(4-bromophenyl)azetidin-2-one (660 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 330 mg of a mixture containing (2'R,3'S)-2',7-(bis)triethylsilyl-3'-desphenyl-3'-(4-bromophenyl) taxol and a small amount of the (2'S,3'R) isomer.

To a solution of 330 mg (0.284 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 265 mg of material which was purified by flash chromatography to give 186 mg (64%) of 3'-desphenyl-3'-(4-bromophenyl) taxol, which was recrystallized from methanol/water.
m.p. 170-172 °C;[α]²⁵_{Na}-50.94° (c 0.01, CHCl₃).
¹H NMR (CDCl₃, 300 MHz) δ 8.12 (d, J = 7.2 Hz, 2H, benzoate ortho), 7.71 (m, 2H, aromatic), 7.61 (m, 1H, aromatic), 7.50-7.47 (m, 6H, aromatic), 7.38 (m, 3H, aromatic), 7.04 (d, J = 8.8 Hz, 1H, NH), 6.27 (s, 1H, H10), 6.23 (dd, J = 8.2, 8.2 Hz, 1H, H13), 5.75 (dd, J = 8.8, 2.2 Hz, 1H, H3'), 5.66 (d, J = 7.1 Hz, 1H, H2β), 4.94 (dd, J = 9.3, 1.7 Hz, 1H, H5), 4.75 (dd, J = 2.2 Hz, 1H, H2'), 4 .38 (m, 1H, H7), 4.29 (d, J = 8.2 Hz, 1H, H20α), 4.18 (d, J = 8.2 Hz, 1H, H20β), 3.79 (d, J = 7.1 Hz, 1H, H3), 3.7 (br, 1H, 2'OH), 2.53 (m, 1H, H6α), 2 .38 (br, 1H, 7OH), 2.37 (s, 3H, 4Ac), 2 .30 (m, 2H, H14), 2. 23 (s, 3H, 10Ac), 1.87 (m, 1H, H6β), 1.80 (br s, 3H, Me18), 1.80 (s, 1H, 1OH), 1.67 (s, 3H, Me19), 1.22 (s, 3H, Me17), 1.13 (s, 3H, Me16).

### EXAMPLE 10

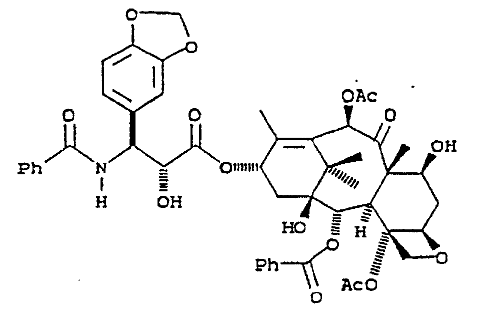

### Preparation of 3'-desphenyl-3'-(3,4-methylene-dioxyphenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(3,4-methylenedioxyphenyl)azetidin-2-one (610 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl-3'-desphenyl-3'-(3,4-methylenedioxyphenyl) taxol and a small amount of the (2'S,3'R) isomer.

To a solution of 320 mg (0.284 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 113 mg of material which was purified by flash chromatography to give 165 mg (64%) of 3-desphenyl-3'-(3,4-methylenedioxyphenyl) taxol, which was recrystallized from methanol/water.
m.p. 178-180 °C;[α]²⁵_{Na}-46.6° (c 0.005, CHCl₃).
¹H NMR (CDCl₃, 300 MHz) δ 8.14 (d, J = 7.2 Hz, 2H, benzoate ortho), 7.72 (m, 2H, aromatic), 7.15 (m, 1H, aromatic), 7.50 (m, 2H, aromatic), 7.38 (m,2H, aromatic), 7.0 (m, 1H, aromatic), 6.94 (m, 2H, aromatic), 6.88 (d, J = 9.1 Hz, 1H, NH), 6.83 (m, 1H, aromatic), 6.28 (s, 1H, H10), 6.23 (dd, J = 9.1, 9.1 Hz, 1H, H13), 5.97 (s, 2H, methylene), 5.69 (dd, J = 9.1, 2.5 Hz, 1H, H3'), 5.68 (d, J = 6.9 Hz, 1H, H2ß), 4.95 (dd, J = 9.6, 2.2 Hz, 1H, H5), 4.72 (dd, J = 2.5 Hz, 1H, H2'), 4.41 (m, 1H, H7), 4.31 (d, J = 8.4 Hz, 1H, H20α), 4.20 (d, J = 8.4 Hz, 1H, H20β), 3.81 (d, J = 6. 9 Hz, 1H, H3), 3.60 (br, 1H, 2'OH), 2.56 (m, 1H, H6α), 2.43 (d, J = 4.1 Hz, 1H, 7OH), 2.39 (s, 3H, 4Ac), 2.31 (m, 2H, H14), 2.24 (s, 3H, 10Ac), 1.88 (m, 1H, H6β), 1.82 (br s, 3H, Me18), 1.69 (s, 1H, 10H), 1.68 (s, 3H, Me19), 1.24 (s, 3H, Me17), 1.15 (s, 3H, Me16).

### EXAMPLE 11

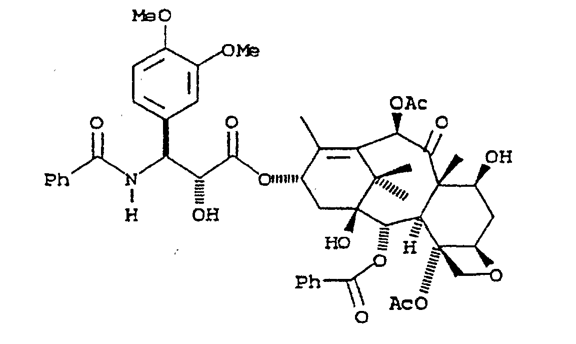

### Preparation of 3'-desphenyl-3'-(3,4-dimethoxyphenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(3,4-dimethoxyphenyl)azetidin-2-one (630 mg, 1.43 mmol) in 2 mL of THF was added dropwise to the mixture. The solution was warmed to 0 °C and kept at that temperature for 1 hr before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 330 mg of a mixture containing (2'R,3'S)-2',7-(bis)triethylsilyl-3'-desphenyl-3'-(3,4-dimethoxyphenyl) taxol and a small amount of the (2'S,3'R) isomer.

To a solution of 330 mg (0.286 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 260 mg of material which was purified by flash chromatography to give 175 mg (67%) of 3'-desphenyl-3'-(3,4-dimethoxyphenyl) taxol, which was recrystallized from methanol/water.
m.p. 165-167 °C;[α]²⁵_{Na}-42.0° (c 0.005, CHCl₃).
¹H NMR (CDCl₃, 300 MHz) δ 8.12 (d, J = 8.3 Hz, 2H, benzoate ortho), 7.73 (d, J = 8.2 Hz, 2H, benzamide ortho), 7.65-7.35 (m, 6H, aromatic), 7.1-7.0 (m, 2H, aromatic), 6.94 (d, J = 8.8 Hz, 1H, NH) , 6.88 (d, J = 8.3 Hz, 2H, aromatic), 6.27 (s, 1H, H10), 6.21 (dd, J = 9.3, 9.3 Hz, 1H, H13), 5.69 (m, 2H, H3, H2β), 4.94 (dd, Hz, J = 9.9, 2.2 Hz, 1H, H5), 4.77 (d, J = 2.8 Hz, 1H, H2'), 4.39 (dd, J = 11.0, 6.6 Hz, 1H, H7), 4.30 (d, J = 8.5 Hz, 1H, H20α), 4.19 (d, J = 8.5 Hz, 1H, H20β), 3.88 (s, 3H, ArOMe), 3.87 (s, 3H, ArOMe), 3.80 (d, J = 7.1 Hz, 1H, H3), 3.59 (d, J = 4.4 Hz, 1H, 2'OH), 2.54 (m, 1H, H6α), 2.38 (s, 3H, 4Ac), 2.36 (m, 2H, H14α, H14β), 2.23 (s, 3H, 10Ac), 1.86 (m, 1H, H6β), 1.80 (br s, 3H, Me18), 1.68 (s, 3H, Me19), 1.23 (s, 3H, Me17), 1.14 (s, 3H, Me16).

### EXAMPLE 12

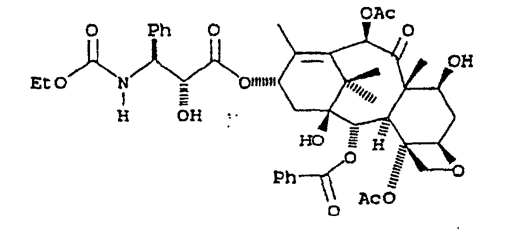

### Preparation of N-debenzoyl-N-ethoxycarbonyl taxol.

To a solution of 7-triethylsilyl baccatin III (155 mg, 0.221 mmol) in 2 mL of THF at -45 °C was added dropwise 0.136 mL of a 1.63M solution of nBuLi in hexane. After 0.5 h at -45 °C, a solution of cis-1-ethoxycarbonyl-3-triethylsilyloxy-4-phenylazetidin-2-one (386 mg, 1.11 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 252 mg of a mixture containing (2'R,3'S)-2',7-(bis)triethylsilyl-N-debenzoyl-N-ethoxycarbonyl taxol and a small amount of the (2'S,3'R) isomer.

To a solution of 252 mg (0.112 mmol) of the mixture obtained from the previous reaction in 12 mL of acetonitrile and 0.6 mL of pyridine at 0 °C was added 1.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 216 mg of material which was purified by flash chromatography to give 155 mg (85%) of N-debenzoyl-N-ethoxycarbonyl taxol, which was recrystallized from methanol/water.
m.p. 161.5-162.5 °C; [α]²⁵_{Na}-62.2° (c 0.51, CHCl₃).
¹H NMR (CDCl₃, 300 MHz) δ 8.12 (d, J = 7.7 Hz, 2H, benzoate ortho), 7.65-7.3 (m, 8H, aromatic), 6.28 (m, 1H, H10) 6.27(m, 1H, H13), 5.67 (d, J = 7.1 Hz, 1H, H2β), 5.53 (d, J = 9.3 Hz, 1H, H3'), 5.29 (d, J = 9.3 Hz, 1H, NH), 4.94 (dd, J = 9.3, 2.2 Hz, 1H, H5), 4.64 (dd, J = 5.0, 2.8 Hz, 1H, H2'), 4.41 (m, 1H, H7), 4.29 (d, J = 8.5 Hz, 1H, H20α), 4.17 (d, J = 8.5 Hz, 1H, H20β), 4.01 (q, J = 7.1 Hz, 2H, COOCH₂CH₃), 3 .79 (d, J = 7.1 Hz, 1H, H3), 3.45 (d, J = 5 Hz, 1H, 2'OH), 2.54 (m, 1H, H6α), 2.47 (d, J = 3.9 Hz 1H, 7OH), 2.36 (s, 3H, 4Ac), 2.24 (s, 3H, 10Ac), 2.22 (m, 2H, H14α, H14β), 1.87 (m, 1H, H6α), 1.83 (br s, 3H, Me18), 1.77. (s, 1H, 10H), 1.68 (s, 3H, Me19), 1.27 (s, 3H, Me17), 1.15 (s, 3H, Me16), 1.14 (t, J = 7.1 Hz, 2H, COOCH₂CH₃).

### EXAMPLE 13

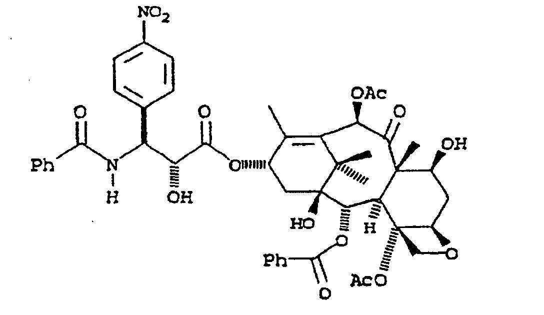

### Preparation of 3'-desphenyl-3'-(4-nitrophenyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of cis-1-benzoyl-3-triethylsilyloxy-4-(4-nitrophenyl)azetidin-2-one (610 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of a mixture containing (2'R,3'S)-2',7-(bis)triethylsilyl-3'-desphenyl-3'-(4-nitrophenyl) taxol and a small amount of the (2'S,3'R) isomer.

To a solution of 320 mg (0.284 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of Lhe ethyl acetate solution gave 255 mg of material which was purified by flash chromatography to give 147 mg (57%) of 3'-desphenyl-3'-(4-nitrophenyl) taxol, which was recrystallized from methanol/water.
m.p. 188-190 °C;[α]²⁵_{Na}-63.7° (c 0.01, CHCl₃).
¹H NMR (CDCl₃, 300 MHz) δ 8.26 (d, J = 8.8 Hz, 2H, benzoate ortho), 8.20 (m, 2H, aromatic), 7.73 (m, 4H, aromatic), 7.60 (m, 1H, aromatic), 7.52 (m, 4H, aromatic), 7.41 (m, 1H, aromatic), 7.15 (d, J = 8.8 Hz, 1H, NH), 6.26 (s, 1H, H10), 6.26 (dd, J = 9.3, 9.3 Hz, 1H, H13), 5.93 (dd, J = 8.8, 2.8 Hz, 1H, H3'), 5.66 (d, J = 6.6 Hz, 1H, H2β), 4.94 (dd, J = 9.3, 1.7 Hz, 1H, H5), 4.82 (dd, J = 3.9, 2.8 Hz, 1H, H2'), 4.38 (m, 1H, H7), 4.30 (d, J = 8.8 Hz, 1H, H20α), 4.19 (d, J = 8.8 Hz, 1H, H20β), 3.86 (d, J = 3 .9 Hz, 1H, 2'OH), 3.79 (d, J = 6.6 Hz, 1H, H3), 2.55 (m, 1H, H6α), 2.46 (d, J = 3.8 Hz, 1H, 7OH), 2.41 (s, 3H, 4Ac), 2.38 (m, 2H, H14), 2.23 (s, 3H, 10Ac), 1.82 (m, 1H, H6β), 1.80 (br s, 3H, Me18), 1.74 (s, 1H, 1OH), 1.68 (s, 3H, Me19), 1.21 (s, 3H, Me17), 1.13 (s, 3H, Me16).

### EXAMPLE 14

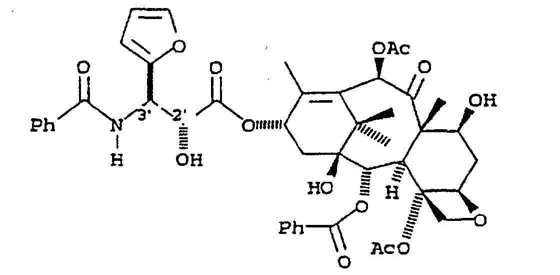

### Preparation of 3'-desphenyl-3'-(2-furyl) taxol.

To a solution of 7-triethylsilyl baccatin III (100 mg, 0.143 mmol) in 1 mL of THF at -45 °C was added dropwise 0.087 mL of a 1.63M solution of nBuLi in hexane. After 0.5 h at -45 °C, a solution of cis-1-benzoyl-3-triethylsilyloxy-4-(2-furyl)azetidin-2-one (266 mg, 0.715 mmol) in 1 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 143 mg of a mixture containing (2'R,3'S)-2',7-(bis)triethylsilyl-3'-desphenyl-3'-(2-furyl) taxol and a small amount of the (2'S,3'R) isomer.

To a solution of 143 mg of the mixture obtained from the previous reaction in 6 mL of acetonitrile and 0.3 mL of pyridine at 0 °C was added 0.9 mL of 48% aqueous HF. The mixture was stirred at 0 °C for 3 h, then at 25 °C for 13 h, and partitioned between saturated aqueous bicarbonate and ethyl acetate. Evaporation of the ethyl acetate solution gave 115 mg of material which was purified by flash chromatography to give 98 mg (81%) of 3'-desphenyl-3'-(2-furyl) taxol, which was recrystallized from methanol/water.
m.p. 174-176 °C;[α]²⁵_{Na}-47.8° (c 0.045, CHCl₃).
¹H NMR (CDCl₃, 300 MHz) δ 8.14 (d, J = 7.0 Hz, 2H, benzoate ortho), 7.74 (m, 2H, aromatic), 7.51 (m, 7H, aromatic), 6.86 (d, J = 9.2 Hz, 1H, NH), 6.40 (d, J = 1.2 Hz, 2H, furyl), 6.29 (s, 1H, H10), 6.24 (dd, J - 9.2, 9.2 Hz, 1H, H13), 5.89 (dd, J = 9.2, 2.4 Hz, 1H, H3'), 5.69 (d, J = 7.0 Hz, 1H, H2β), 4.96 (dd, J = 9.5, 1.8 Hz, 1H, H5), 4.83 (d, J = 2.4 Hz, 1H, H2'), 4.42 (dd, J = 10.7, 6.7 Hz, 1H, H7), 4.31 (d, J = 8.6 Hz, 1H, H20α), 4.20 (d, J = 8.6 Hz, 1H, H20β), 3.83 (d, J = 7.0 Hz, 1H, H3), 2.56 (m, 1H, H6α), 2.43 (s, 3H, 4Ac), 2.35 (m, 2H, H14), 2.24 (s, 3H, 10Ac), 1.89 (m, 1H, H6β), 1.87 (br s, 3H, Me18), 1.87 (s, 1H, 10H), 1.69 (s, 3H, Me19), 1.25 (s, 3H, Me17), 1.15 (s, 3H, Me16).

### EXAMPLE 15

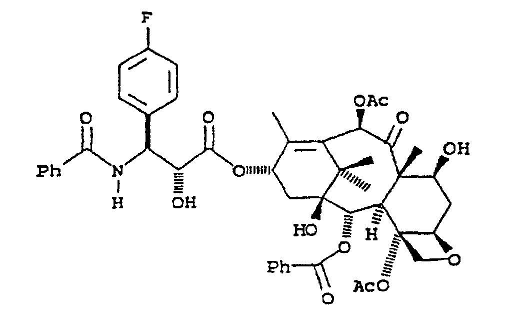

### Preparation of 3'-desphenyl-3'-(4-fluorophenyl) taxol.

To a solution of 7-triethylsilyl baccatin III (200 mg, 0.286 mmol) in 2 mL of THF at -45 °C was added dropwise 0.174 mL of a 1. 63M solution of nBuLi in hexane. After 0.5 h at -45 °C, a solution of cis-1-benzoyl-3-triethylsilyloxy-4-(4-fluorophenyl)azetidin-2-one (570 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 315 mg of a mixture containing (2'R,3'S)-2',7-(bis)triethylsilyl-3'-desphenyl-3'-(4-fluorophenyl) taxol and a small amount of the (2'S,3'R) isomer.

To a solution of 315 mg (0.286 mmol) of the mixture obtained from the previous reaction in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 250 mg of material which was purified by flash chromatography to give 160 mg (64%) of 3'-desphenyl-3'-(4-fluorophenyl) taxol, which was recrystallized from methanol/water.
m.p.171-173 °C;[α]²⁵_{Na}-49.0° (c 0.005, CHCl₃).
¹H NMR (CDCl₃, 300 MHz) δ 8.13 (d, J = 7.5 Hz, 2H, benzoate ortho), 7.25 (m, 2H, aromatic), 7.61 (m, 1H, aromatic), 7.50 (m, 4H, aromatic), 7.43 (m, 2H, aromatic), 7.10 (m, 2H, aromatic), 6.96 (d, J = 8.7 Hz, 1H, NH) , 6.27 (s, 1H, H10), 6.25 (dd, J = 8.7, 8.7 Hz, 1H, H13), 5.79 (dd, J = 8.7, 2.4 Hz, 1H, H3'), 5.67 (d, J = 7.1 Hz, 1H, H2ß), 4.45 (dd, J = 7.9 Hz, 1H, H5), 4.76 (dd, J = 4.8, 2.4 Hz, 1H, H2'), 4.39 (m, 1H, H7), 4.31 (d, J = 8.9 Hz, 1H, H20α), 4. 20 (d, J = 8.9 Hz, 1H, H20β), 3.80 (d, J = 7.1 Hz, 1H, H3), 3 .57 (d, J = 4.8 Hz, 1H, 2'OH) , 2.58 (m, 1H, H6a), 2.43 (d, J = 4.3 Hz, 1H, 7OH), 2.38 (s, 3H, 4Ac), 2.30 (m, 2H, H14), 2.24 (s, 3H, 10Ac), 1.85 (m, 1H, H6β), 1.80 (br s, 3H, Me18), 1.69 (s, 1H, 10H), 1.55 (s, 3H, Me19), 1.23 (s, 3H, Me17), 1.14 (s, 3H, Me16).

### EXAMPLE 16

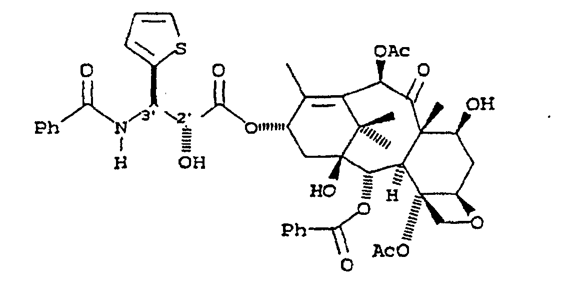

### Preparation of 3'-Desphenyl-3'-(2-thienyl) taxol.

To a solution of 7-triethylsilyl baccatin III (100 mg, 0.143 mmol) in 1 mL of THF at -45 °C was added dropwise 0.087 mL of a 1.63M solution of nBuLi in hexane. After 0.5 h at -45 °C, a solution of cis-1-(4-benzoyl)-3-triethylsilyloxy-4-(2-thienyl)azetidin-2-one (277 mg, 0.715 mmol) in 1 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 169 mg of a mixture containing (2'R,3'S)-2',7-(bis)triethylsilyl-3'-desphenyl-3'-(2-thienyl) taxol and a small amount of the (2'S,3'R) isomer.

To a solution of 169 mg of the mixture obtained from the previous reaction in 6 mL of acetonitrile and 0.3 mL of pyridine at 0 °C was added 0.9 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 140 mg of material which was purified by flash chromatography to give 93 mg (76%) of 3'-desphenyl-3'-(2-thienyl) taxol, which was recrystallized from methanol/water.
m.p. 173-175 °C; [α]²⁵_{Na}-42.1° (c 0.515, CHCl₃).
¹H NMR (CDCl₃, 300 MHz) δ 8.14 (d, J = 7.1 Hz, 2H, benzoate ortho), 7.72 (d, J = 8.7 Hz, 2H, benzamide ortho), 7.65-7.35 (m, 6H, aromatic), 7.31 (dd, J = 5.5, 1.1 Hz, 1H, thienyl), 7.19 (dd, J =3.9, 1.1 Hz, 1H, thienyl), 7.03 (dd, J = 5.5, 3.9 Hz, 1H, thienyl), 6.96 (d, J = 8.8 Hz, 1H, NH), 6.28 (s, 1H, H10), 6.24 (dd, J = 8.8, 7.7 Hz, 1H, H13), 6.05 (dd, J = 8.8, 1.7 Hz, 1H, H3'), 5.68 (d, J = 7.1 Hz, 1H, H2), 4.95 (dd, J = 9.3, 1.7 Hz, 1H, H5), 4.78 (d, J = 2.2 Hz, 1H, H2'), 4 .40 (dd, J = 11.0, 6 .6 Hz, 1H, H7), 4.31 (d, J = 8. 5 Hz, 1H, H20α), 4 .20 (d, J = 8.5 Hz, 1H, H20β), 3.81 (d, J = 7.1 Hz, 1H, H3), 3.72 (br. s, 1H, 2'OH), 2.54 (m, 1H, H6α), 2.41 (s, 3H, 4Ac), 2.37 (m, 2H, H14α, H14β), 2.23 (s, 3H, 10Ac), 1.88 (m, 1H, H6α), 1.82 (br s, 3H, Me18), 1.68 (s, 3H, Me19), 1.23 (s, 3H, Me17), 1.14 (s, 3H, Me16).

### EXAMPLE 17

### Preparation of 2',7-hydroxy protected Taxol using magnesium alkoxide:

To a solution of 7-triethylsilyl baccatin III (100 mg, 0.143 mmol) in 1 mL of THF at -45 °C was added dropwise 0.048 mL of a 3.0 M solution of methyl magnesium bromide in ether. After 1 h at -45 °C, a solution of (+)-cis-1-benzoyl-3-triethylsilyloxy-4-phenylazetidin-2-one (82 mg, 0.215 mmol) in 1 mL of THF was added dropwise to the mixture. The solution was warmed to 0 °C and kept at that temperature for 4 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by flash chromatography followed by recrystallization to give 148 mg (96%) of (2'R,3'S)-2',7-(bis)triethylsilyl taxol.

### EXAMPLE 18

### Preparation of 2',7-hydroxy protected Taxol using potassium alkoxide:

To a solution of 7-triethylsilyl baccatin III (100 mg, 0.143 mmol) in 1 mL of THF at -45°C was added dropwise 0.286 mL of a 0.5 M solution of potassium hexamethyldisilazide in toluene. After 1 h at -45 °C, a solution of (+)-cis-1-benzoyl-3-triethylsilyloxy-4-phenylazetidin-2-one (82 mg, 0.215 mmol) in 1 mL of THF was added dropwise to the mixture. The solution was warmed to 0°C and kept at that temperature for 3 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by flash chromatography followed by recrystallization to give 139 mg (90%) of (2'R,3'S)-2',7-(bis)triethylsilyl taxol.

### EXAMPLE 19

### Preparation of 2',7-hydroxy protected Taxol using lithium alkoxide from lithium hexamethyldisilazide:

To a solution of 7-triethylsilyl baccatin III (100 mg, 0.143 mmol) in 1 mL of THF at -45 °C was added dropwise 0.143 mL of a 1.0 M solution of lithium hexamethyldisilazide in THF. After 1 h at -45 °C, a solution of (+)-cis-1-benzoyl-3-triethylsilyloxy-4-phenylazetidin-2-one (82 mg, 0.215 mmol) in 1 mL of THF was added dropwise to the mixture. The solution was warmed to 0°C and kept at that temperature for 2 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by flash chromatography followed by recrystallization to give 151 mg (98%) of (2'R,3'S)-2',7-(bis)triethylsilyl taxol.

### EXAMPLE 20

### Preparation of Taxol using lithium alkoxide (from lithium hexamethyldisilazide):

To a solution of 7-triethylsilyl baccatin III (100 mg, 0.143 mmol) in 1 mL of THF at -45 °C was added dropwise 0.143 mL of a 1.0 M solution of lithium hexamethyldisilazide in THF. After 1 h at -45 °C, a solution of (+)-cis-1-benzoyl-3- (2-methoxy-2-propyloxy)-4-phenylazetidin-2-one (58 mg, 0.172 mmol) in 1 mL of THF was added dropwise to the mixture. The solution was warmed to 0°C and kept at that temperature for 2 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by recrystallization to give 147 mg (99%) of (2'R,3'S)-2'-(2-methoxy-2-propyloxy)-7-triethylsilyl taxol.

To a solution of 116 mg (0.112 mmol) of (2'R,3'S)-2'-(2-methoxy-2-propyloxy)-7-triethylsilyl taxol in 6 mL of acetonitrile and 0.3 mL of pyridine at 0°C was added 0.9 mL of 48% aqueous HF. The mixture was stirred at 0°C for 8 h, then at 25°C for 10 h. The mixture was partitioned between saturated aqueous sodium bicarbonate and ethyl acetate. Evaporation of the ethyl acetate solution gave 113 mg of material which was purified by recrystallization to give 95 mg (99%) of taxol, which was identical with an authentic sample in all respects.

### EXAMPLE 21

### Preparation of 2',7-hydroxy protected Taxol using sodium alkoxide:

To a solution of 7-triethylsilyl baccatin III (100 mg, 0.143 mmol) in 1 mL of THF at -45°C is added dropwise 0.143 mL of a 1 M solution of sodium hexamethyldisilazide in THF. After 1 h at -45 °C, a solution of (+)-cis-1-benzoyl-3-triethylsilyloxy-4-phenylazetidin-2-one (82 mg, 0.215 mmol) in 1 mL of THF is added dropwise to the mixture. The solution is warmed to 0°C and kept at that temperature for 3 h before 1 mL of a 10% solution of AcOH in THF is added. The mixture is partitioned between saturated aqueous NaHCO₃ and 60/40 ethyl acetate/hexane. Evaporation of the organic layer gives a residue which is purified by flash chromatography followed by recrystallization to give 108 mg (70%) of (2'R,3'S)-2',7-(bis)triethylsilyl taxol.

### EXAMPLE 22

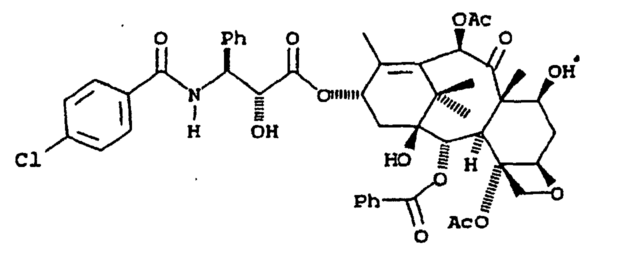

### Preparation of N-debenzoyl-N-(4-chlorobenzoyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of (+)-cis-1-(4-chlorobenzoyl)-3-triethylsilyloxy-4-phenylazetidin-2-one (215 mg, 0.515 mmol) in 2 mL of THF was added dropwise to the mixture. The solution was warmed to 0 °C and kept at that temperature for 2 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 320 mg of crude (2'R,3'S)-2',7-(bis)triethylsilyl-N-debenzoyl-N-(4-chlorobenzoyl) taxol.

To a solution of 320 mg (0.286 mmol) of this crude product in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 252 mg of material which was purified by flash chromatography to give 213 mg (84%) of N-debenzoyl-N-(4-chlorobenzoyl) taxol, which was recrystallized from methanol/water.
m.p. 179-181 °C; [α]²⁵_{Na}-49.8° (c 0.01, CHCl₃).
¹H NMR (CDCl₃, 300 MHz) δ 8.12 (d, J = 7.1 Hz, 2H, benzoate ortho), 7.64 (m, 2H, aromatic), 7.60 (m, 1H, aromatic), 7.49 (m, 9H, aromatic), 7.03 (d, J = 8.8 Hz, 1H, NH), 6.26 (s, 1H, H10), 6.21 (dd, J = 8.2, 8.2 Hz, 1H, H13), 5.76 (dd, J = 8. 8, 2 .2 Hz, 1H, H3'), 5. 66 (d, J = 7.1 Hz, 1H, H2β), 4.92 (dd, J = 9.9, 1.1 Hz, 1H, H5), 4 .77 (dd, J = 5.5, 2 .2 Hz, 1H, H2'), 4 .38 (m, 1H, H7), 4 .29 (d, J = 8.8 Hz, 1H, H20α), 4.18 (d, J = 8.5 Hz, 1H, H20β), 3.78 (d, J = 6.6 Hz, 1H, H3), 3.35 (d, J = 5.5 Hz, 1H, 2'OH), 2.55 (m, 1H, H6α), 2.49 (d, J = 4.2 Hz, 1H, 7OH), 2.36 (s, 3H, 4Ac), 2 .28 (m, 2H, H14), 2.22 (s, 3H, 10Ac), 1.85 (m, 1H, H6β), 1.77 (br s, 3H, Me18), 1.76 (s, 1H, 1OH), 1.67 (s, 3H, Me19), 1.22 (s, 3H, Me17), 1.13 (s, 3H, Me16).

### EXAMPLE 23

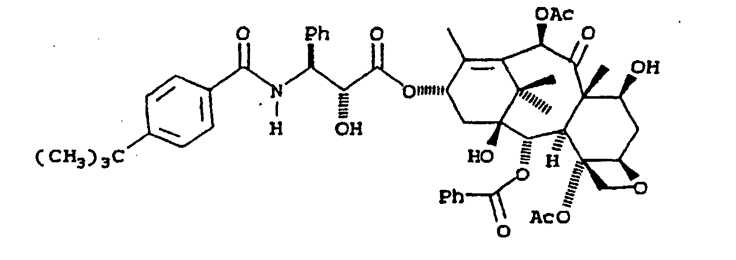

### Preparation of N-debenzoyl-N-(4-t-butylbenzoyl) taxol.

To a solution of 7-triethylsilyl baccatin III (200 mg, 0.286 mmol) in 2 mL of THF at -45 °C was added dropwise 0.174 mL of a 1.63M solution of nBuLi in hexane. After 0.5 h at -45 °C, a solution of (+)-cis-1-(4-t-butylbenzoyl)-3-triethylsilyloxy-4-phenylazetidin-2-one (226 mg, 0.515 mmol) in 2 mL of THF was added dropwise to the mixture. The solution was warmed to 0 °C and kept at that temperature for 2 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 330 mg of crude (2'R,3'S)-2',7-(bis)triethylsilyl-N-debenzoyl-N-(4-t-butylbenzoyl) taxol.

To a solution of 330 mg (0.289 mmol) of this crude product in 18 mL of acetonitrile and 0.93 mL of pyridine at 0 °C was added 2.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 260 mg of material which was purified by flash chromatography to give 240 mg (92%) of N-debenzoyl-N-(4-t-butylbenzoyl) taxol, which was recrystallized from methanol/water.
m.p. 171-173 °C; [α]²⁵_{Na}-49.1° (c 0.05, CHCl₃).
¹H NMR (CDCl₃, 300 MHz) δ 8.13 (d, J = 7.1 Hz, 2H, benzoate ortho), 7.76-7.25 (m, 12H, aromatic), 6.98 (d, J = 8.8 Hz, 1H, NH), 6.27 (s, 1H, H10), 6.21 (dd, J = 8.8, 8.8 Hz, 1H, H13), 5.77 (dd, J = 8.8, 2.7 Hz, 1H, H3'), 5.67 (d, J = 6.6 Hz, 1H, H2β), 4.94 (dd, J = 9.3, 1.2 Hz, 1H, H5), 4.78 (dd, J = 4.4, 2.7 Hz, 1H, H2'), 4 .38 (m, 1H, H7), 4 .29 (d, J = 8.2 Hz, 1H, H20α), 4.20 (d, J = 8.2 Hz, 1H, H20β), 3.79 (d, J = 6.6 Hz, 1H, H3), 3.65 (d, J = 4.4 Hz, 1H, 2'OH), 2.57 (m, 1H, H6α), 2.48 (d, J = 4.1 Hz, 1H, 7OH), 2.37 (s, 3H, 4Ac), 2.31 (m, 2H, H14), 2.22 (s, 3H, 10Ac), 1.85 (m, 1H, H6β), 1.79 (br s, 3H, Me18), 1.68 (s, 1H, 1OH), 1.68 (s, 3H, Me19), 1.29 (s, 9H, Ar^{t}Bu), 1.23 (s, 3H, Me17), 1.13 (s, 3H, Me16).

### EXAMPLE 24

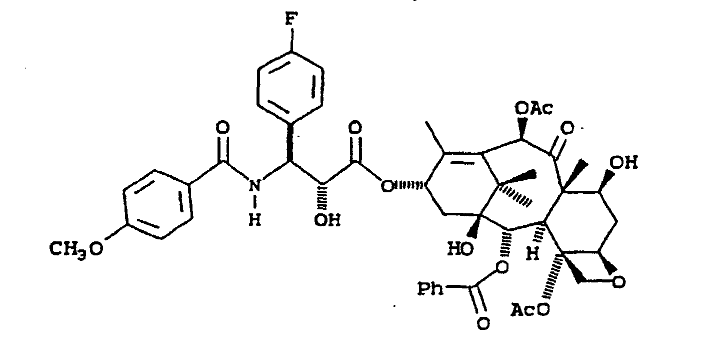

### Preparation of N-debenzoyl-N-(4-methoxybenzoyl)-3'-desphenyl-3'-(4-fluorophenyl) taxol.

To a solution of 7-triethylsilyl baccatin III (200 mg, 0.285 mmol) in 2 mL of THF at -45 °C was added dropwise 0.175 mL of a 1.63M solution of nBuLi in hexane. After 0.5 h at -45 °C, a solution of cis-1-(4-methoxybenzoyl)-3-triethylsilyloxy-4-(4-fluorophenyl)azetidin-2-one (614 mg, 1.43 mmol) in 2 mL of THF was added dropwise to the mixture. The solution was warmed to 0 °C and kept at that temperature for 1 h before 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 362 mg of a mixture containing (2'R,3'S)-2', 7-(bis)triethylsilyl-N-debenzoyl-N-(4-methoxybenzoyl)-3'-desphenyl-3'-(4-fluorophenyl) taxol and a small amount of the (2'S,3'R) isomer.

To a solution of 362 mg of the mixture obtained from the previous reaction in 12 mL of acetonitrile and 0.6 mL of pyridine at 0 °C was added 1.8 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 269 mg of material which was purified by flash chromatography to give 183 mg (71%) of N-debenzoyl-N-(4-methoxybenzoyl)-3'-desphenyl-3'-(4-fluorophenyl) taxol, which was recrystallized from methanol/water.
m.p. 172.5-174.5 °C;[α]²⁵_{Na}-47.0° (c 0.0044, CHCl₃).
¹H NMR (CDCl₃, 300 MHz) δ 8.13 (d, J = 7.2 Hz, 2H, benzoate ortho), 7.7-7.4 (m, 9H, aromatic), 7.10 (dd, J = 8.8, 8.8 Hz, 2H, aromatic), 6.97 (d, J = 8.8 Hz, 1H, NH), 6.27 (s, 1H, H10), 6.23 (dd, J = 8.8, 8.8 Hz, 1H, H13), 5.76 (dd, J = 8.8, 2.2 Hz, 1H, H3'), 5.67 (d, J = 7.1 Hz, 1H, H2β), 4.94 (dd, J = 9.9, 2.2 Hz, 1H, H5), 4.75 (dd, J - 4.4, 2.2 Hz, 1H, H2'), 4 .39 (m, 1H, H7), 4.31 (d, J = 8.5 Hz, 1H, H20α), 4.19 (d, J = 8.5 Hz, 1H, H20β), 3.79 (d, J = 7.1 Hz, 1H, H3), 3.59 (d, J = 4.4 Hz, 1H, 2'OH), 2.54 (m, 1H, H6α), 2.47 (d, J = 4.4 Hz, 1H, 7OH), 2.36 (s, 3H, 4Ac), 2.30 (m, 2H, H14α, H14β), 2.24 (s, 3H, 10Ac), 1.88 (m, 1H, H6α), 1.78 (br s, 3H, Me18), 1.74 (s, 1H, 1OH), 1.68 (s, 3H, Me19), 1.23 (s, 3H, Me17), 1.14 (s, 3H, Me16).

In view of the above, it will be seen that the several objects of the invention are achieved.

As various changes could be made in the above compositions and processes without departing from the scope of the invention, it is intended that all matter contained in the above description be interpreted as illustrative and not in a limiting sense.

## Claims (Claims for the following Contracting State(s): AT, BE, CH, LI, DE, DK, FR, GB, GR, IE, IT, MC, NL, SE)

1. A metal alkoxide having the formula: 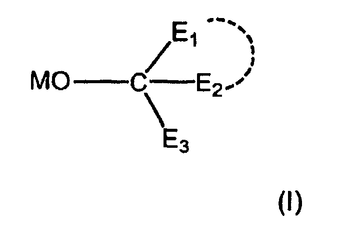 wherein
M is a metal;
E₁, E₂ and the carbon to which they are attached comprise a bi-, tri- or tetracyclic taxane nucleus, and
E₃ is hydrogen.

2. A metal alkoxide having the formula: 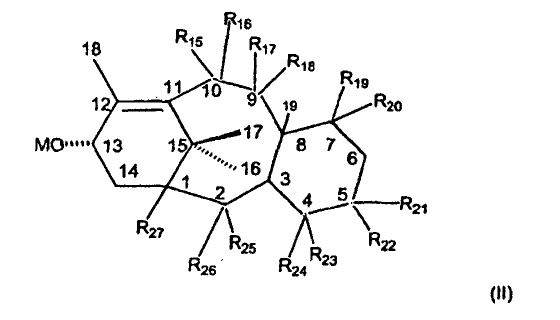 wherein
M is a metal;
R₁₅ and R₁₆ are independently hydrogen, hydroxy, lower alkanoyloxy, alkenoyloxy, alkynoyloxy, aryloyloxy or R₁₅ and R₁₆ together form an oxo;
R₁₇ and R₁₈ are independently hydrogen or lower alkanoyloxy, alkenoyloxy, alkynoyloxy, or aryloyloxy or R₁₇ and R₁₈ together form an oxo;
R₁₉ and R₂₀ are independently hydrogen or hydroxy or lower alkanoyloxy, alkenoyloxy, alkynoyloxy, or aryloyloxy;
R₂₁ and R₂₂ are independently hydrogen or lower alkanoyloxy, alkenoyloxy, alkynoyloxy, or aryloyloxy or R₂₁ and R₂₂ together form an oxo;
R₂₃ and R₂₄ are independently hydrogen or hydroxy or lower alkanoyloxy, alkenoyloxy, alkynoyloxy, or aryloyloxy or
R₂₃ and R₂₄ together form an oxo or methylene or
R₂₃ and R₂₄ together with the carbon atom to which they are attached form an oxirane ring or
R₂₃ and R₂₂ together with the carbon atom to which they are attached form an oxetane ring;
R₂₅ is hydrogen, hydroxy, or lower alkanoyloxy, alkenoyloxy, alkynoyloxy, or aryloyloxy;
R₂₆ is hydrogen, hydroxy, or lower alkanoyloxy, alkenoyloxy, alkynoyloxy, or aryloyloxy; or
R₂₅ and R₂₆ taken together form an oxo; and
R₂₇ is hydrogen, hydroxy or lower alkoxy, alkanoyloxy, alkenoyloxy, alkynoyloxy, or aryloyloxy.

3. A metal alkoxide according to claim 2, wherein R₂₂ and R₂₃ together with the carbon atoms to which they are attached form an oxetane ring.

4. A metal alkoxide according to claim 2 or claim 3,
wherein
R₁₅ is -OT₂ or -OCOCH₃;
R₁₆ is hydrogen;
R₁₇ and R₁₈ together form an oxo;
R₁₉ is -OT₁;
R₂₄ and R₂₁ are hydrogen;
R₂₂ and R₂₃ and the carbons to which they are attached form an oxetane ring;
R₂₄ is -OCOCH₃;
R₂₅ is PhCOO-;
R₂₆ is hydrogen;
R₂₇ is hydroxy; and
T₁ and T₂ are independently hydrogen or hydroxy protecting groups.

5. A metal alkoxide according to any one of claims 1 to 4, wherein M is a Group IA, IIA, IIIA, lanthanide, actinide, or a transition Group IIIA, IVA, VA, or VIA metal.

6. A metal alkoxide according to any one of claims 1 to 5, wherein the metal is lithium, magnesium, sodium, potassium or titanium.

7. A metal alkoxide according to claim 6, where in the metal is lithium.

## Claims (Claims for the following Contracting State(s): ES)

1. A process for the production of a metal alkoxide comprising reacting an alcohol with an organometallic compound wherein the metal alkoxide has the formula: 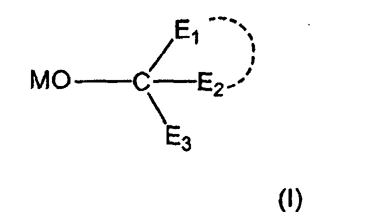 wherein
M is a metal;
E₁, E₂ and the carbon to which they are attached comprise a bi-, tri- or tetracyclic taxane nucleus, and
E₃ is hydrogen.

2. A process for the production of a metal alkoxide comprising reacting an alcohol with an organometallic compound wherein the metal alkoxide has the formula: 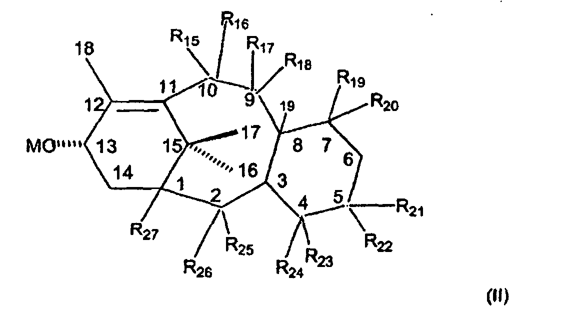 wherein
M is a metal;
R₁₅ and R₁₆ are independently hydrogen, hydroxy, lower alkanoyloxy, alkenoyloxy, alkynoyloxy, aryloyloxy or R₁₅ and R₁₆ together form an oxo;
R₁₇ and R₁₈ are independently hydrogen or lower alkanoyloxy, alkenoyloxy, alkynoyloxy, or aryloyloxy or R₁₇ and R₁₈ together form an oxo;
R₁₉ and R₂₀ are independently hydrogen or hydroxy or lower alkanoyloxy, alkenoyloxy, alkynoyloxy, or aryloyloxy;
R₂₁ and R₂₂ are independently hydrogen or lower alkanoyloxy, alkenoyloxy, alkynoyloxy, or aryloyloxy or R₂₁ and R₂₂ together form an oxo;
R₂₃ and R₂₄ are independently hydrogen or hydroxy or lower alkanoyloxy, alkenoyloxy, alkynoyloxy, or aryloyloxy or
R₂₃ and R₂₄ together form an oxo or methylene or
R₂₃ and R₂₄ together with the carbon atom to which they are attached form an oxirane ring or
R₂₃ and R₂₂ together with the carbon atom to which they are attached form an oxetane ring;
R₂₅ is hydrogen, hydroxy, or lower alkanoyloxy, alkenoyloxy, alkynoyloxy, or aryloyloxy;
R₂₆ is hydrogen, hydroxy, or lower alkanoyloxy, alkenoyloxy, alkynoyloxy, or aryloyloxy; or
R₂₅ and R₂₆ taken together form an oxo; and
R₂₇ is hydrogen, hydroxy or lower alkoxy, alkanoyloxy, alkenoyloxy, alkynoyloxy, or aryloyloxy.

3. A process according to claim 2, wherein R₂₂ and R₂₃ together with the carbon atoms to which they are attached form an oxetane ring.

4. A process according to claim 2 or claim 3, wherein
R₁₅ is -OT₂ or -OCOCH₃;
R₁₆ is hydrogen;
R₁₇ and R₁₈ together form an oxo;
R₁₉ is OT₁;
R₂₀ and R₂₁ are hydrogen;
R₂₂ and R₂₃ and the carbons to which they are attached form an oxetane ring;
R₂₄ is -OCOCH₃;
R₂₅ is PhCOO-;
R₂₆ is hydrogen;
R₂₇ is hydroxy; and
T₁ and T₂ are independently hydrogen or hydroxy protecting groups.

5. A process according to any one of claims 1 to 4, wherein M is a Group IA, IIA, IIIA, lanthanide, actinide, or a transition Group IIIA, IVA, VA, or VIA metal.

6. A process according to any one of claims 1 to 5, wherein the metal is lithium, magnesium, sodium, potassium or titanium.

7. A process according to claim 6, wherein the metal is lithium.

## Patentansprüche (Patentansprüche für folgende(n) Vertragsstaat(en): AT, BE, CH, LI, DE, DK, FR, GB, GR, IE, IT, LU, MC, NL, SE)

1. Metallalkoxid mit der Formel 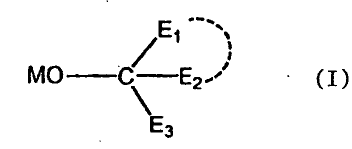 worin
M ein Metall ist,
E₁, E₂ und der Kohlenstoff, an dem sie hängen, einen bi-, tri- oder tetracyclischen Taxankern bilden und
E₃ Wasserstoff ist.

2. Metallalkoxid mit der Formel 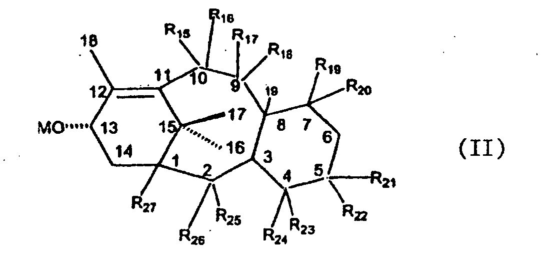 worin
M ein Metall ist,
R₁₅ und R₁₆ unabhängig Wasserstoff, Hydroxy, niederes Alkanoyloxy, Alkenoyloxy, Alkynoyloxy, Aryloyloxy sind oder R₁₅ und R₁₆ zusammen ein Oxo bilden,
R₁₇ und R₁₈ unabhängig Wasserstoff oder niederes Alkanoyloxy, Alkenoyloxy, Alkynoyloxy oder Aryloyloxy sind oder R₁₇ und R₁₈ zusammen ein Oxo bilden,
R₁₉ und R₂₀ unabhängig Wasserstoff oder Hydroxy oder niederes. Alkanoyloxy, Alkenoyloxy, Alkynoyloxy oder Aryloyloxy sind,
R₂₁ und R₂₂ unabhängig Wasserstoff oder niederes Alkanoyloxy, Alkenoyloxy, Alkynoyloxy oder Aryloyloxy sind oder R₂₁ und R₂₂ zusammen ein Oxo bilden,
R₂₃ und R₂₄ unabhängig Wasserstoff oder Hydroxy oder niederes Alkanoyloxy, Alkenoyloxy, Alkynoyloxy oder Aryloyloxy sind oder
R₂₃ und R₂₄ zusammen ein Oxo oder Methylen bilden oder
R₂₃ und R₂₄ zusammen mit dem Kohlenstoffatom, an dem sie hängen, einen Oxiranring bilden oder
R₂₃ und R₂₂ zusammen mit dem Kohlenstoffatom, an dem sie hängen, einen Oxetanring bilden,
R₂₅ Wasserstoff, Hydroxy oder niederes Alkanoyloxy, Alkenoyloxy, Alkynoyloxy oder Aryloyloxy ist,
R₂₆ Wasserstoff, Hydroxy oder niederes Alkanoyloxy, Alkenoyloxy, Alkynoyloxy oder Aryloyloxy ist, oder
R₂₅ und R₂₆ zusammen ein Oxo bilden und
R₂₇ Wasserstoff, Hydroxy oder niederes Alkoxy, Alkanoyloxy, Alkenoyloxy, Alkynoyloxy oder Aryloyloxy ist

3. Metallalkoxid nach Anspruch 2, bei dem R₂₂ und R₂₃ zusammen mit dem Kohlenstoffatom, an dem sie hängen, einen Oxetanring bilden.

4. Metallalkoxid nach Anspruch 2 oder Anspruch 3, bei dem
R₁₅ -OT₂ oder_{\} -OCOCH₃ ist,
R₁₆ Wasserstoff ist,
R₁₇ und R₁₈ zusammen ein Oxo bilden,
R₁₉ -OT₁ ist,
R₂₀ und R₂₁ Wasserstoff sind,
R₂₂ und R₂₃ und die Kohlenstoffatome, an denen sie hängen, einen Oxetanring bilden,
R₂₄ -OCOCH₃ ist,
R₂₅ PhCOO- ist,
R₂₆ Wasserstoff ist,
R₂₇ Hydroxy ist, und
T₁ und T₂ unabhängig Wasserstoff oder Hydroxy-Schutzgruppen sind.

5. Metallalkoxid nach einem der Ansprüche 1 bis 4, bei dem M ein Metall der Gruppe IA, IIA, IIIA, ein Lanthaniden- oder Actinidenmetall oder ein Metall der Übergangsgruppe IIIA, IVA, VA oder VIA ist.

6. Metallalkoxid nach einem der Ansprüche 1 bis 5, bei dem das Metall Lithium, Magnesium, Natrium, Kalium oder Titan ist.

7. Metallalkoxid nach Anspruch 6, bei dem das Metall Lithium ist.

## Patentansprüche (Patentansprüche für folgende(n) Vertragsstaat(en): ES)

1. Verfahren zur Herstellung eines Metallalkoxids durch Umsetzen eines Alkohols mit einer organometallischen Verbindung, bei dem das Metallalkoxid die Formel 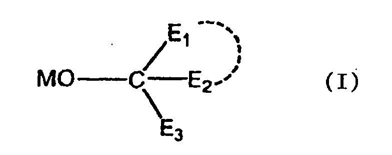 hat, worin
M ein Metall ist,
E₁, E₂ und der Kohlenstoff, an dem sie hängen, einen bi-, tri- oder tetracyclischen Taxankern bilden und
E₃ Wasserstoff ist.

2. Verfahren zur Herstellung eines Metallalkoxids durch Umsetzen eines Alkohols mit einer organometallischen Verbindung, bei dem das Metallalkoxid die Formel 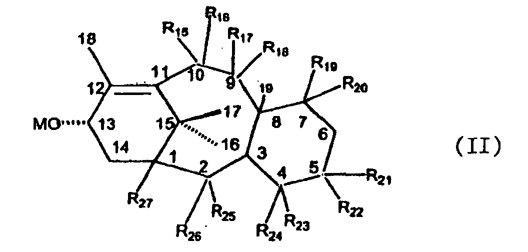 hat, worin
M ein Metall ist,
R₁₅ und R₁₆ unabhängig Wasserstoff, Hydroxy, niederes Alkanoyloxy, Alkenoyloxy, Alkynoyloxy, Aryloyloxy sind oder R₁₅ und R₁₆ zusammen ein Oxo bilden,
R₁₇ und R₁₈ unabhängig Wasserstoff oder niederes Alkanoyloxy, Alkenoyloxy, Alkynoyloxy oder Aryloyloxy sind oder R₁₇ und R₁₈ zusammen ein Oxo bilden,
R₁₉ und R₂₀ unabhängig Wasserstoff oder Hydroxy oder niederes Alkanoyloxy, Alkenoyloxy, Alkynoyloxy oder Aryloyloxy sind,
R₂₁ und R₂₂ unabhängig Wasserstoff oder niederes Alkanoyloxy, Alkenoyloxy, Alkynoyloxy oder Aryloyloxy sind oder R₂₁ und R₂₂ zusammen ein Oxo bilden,
R₂₃ und R₂₄ unabhängig Wasserstoff oder Hydroxy oder niederes Alkanoyloxy, Alkenoyloxy, Alkynoyloxy oder Aryloyloxy sind oder
R₂₃ und R₂₄ zusammen ein Oxo oder Methylen bilden oder
R₂₃ und R₂₄ zusammen mit dem Kohlenstoffatom, an dem sie hängen, einen Oxiranring bilden oder
R₂₃ und R₂₂ zusammen mit dem Kohlenstoffatom, an dem sie hängen, einen Oxetanring bilden,
R₂₅ Wasserstoff, Hydroxy oder niederes Alkanoyloxy, Alkenoyloxy, Alkynoyloxy oder Aryloyloxy ist,
R₂₆ Wasserstoff, Hydroxy oder niederes Alkanoyloxy, Alkenoyloxy, Alkynoyloxy oder Aryloyloxy ist, oder
R₂₅ und R₂₆ zusammen ein Oxo bilden und
R₂₇ Wasserstoff, Hydroxy oder niederes Alkoxy, Alkanoyloxy, Alkenoyloxy, Alkynoyloxy oder Aryloyloxy ist

3. Verfahren nach Anspruch 2, bei dem R₂₂ und R₂₃ zusammen mit dem Kohlenstoffatom, an dem sie hängen, einen Oxetanring bilden.

4. Verfahren nach Anspruch 2 oder Anspruch 3, bei dem
R₁₅ -OT₂ oder -OCOCH₃ ist,
R₁₆ Wasserstoff ist,
R₁₇ und R₁₈ zusammen ein Oxo bilden,
R₁₉ -OT₁ ist,
R₂₀ und R₂₁ Wasserstoff sind,
R₂₂ und R₂₃ und die Kohlenstoffatome, an denen sie hängen, einen Oxetanring bilden,
R₂₄ -OCOCH₃ ist,
R₂₅ PhCOO- ist,
R₂₆ Wasserstoff ist,
R₂₇ Hydroxy ist, und
T₁ und T₂ unabhängig Wasserstoff oder Hydroxy-Schutzgruppen sind.

5. Verfahren nach einem der Ansprüche 1 bis 4, bei dem M ein Metall der Gruppe IA, IIA, IIIA, ein Lanthaniden- oder Actinidenmetall oder ein Metall der Übergangsgruppe IIIA, IVA, VA oder VIA ist.

6. Verfahren nach einem nach einem der Ansprüche 1 bis 5, bei dem das Metall Lithium, Magnesium, Natrium, Kalium oder Titan ist.

7. Verfahren nach Anspruch 6, bei dem das Metall Lithium ist.

## Revendications (Revendications pour l'(les) Etat(s) contractant(s) suivant(s): AT, BE, CH, LI, DE, DK, FR, GB, GR, IE, IT, LU, MC, NL, SE)

1. Alcoxyde de métal répondant à la formule : 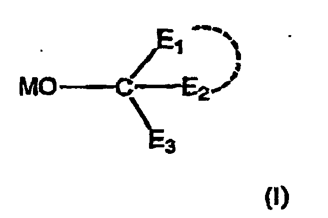 dans laquelle
M est un métal ;
E₁, E₂ et l'atome de carbone sur lequel ils sont fixés comprennent un noyau taxane bi-, tri- ou tétracyclique, et
E₃ est un atome d'hydrogène.

2. Alcoxyde de métal répondant à la formule : 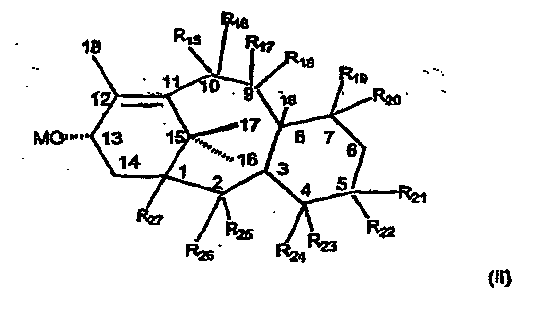 dans laquelle
M est un métal ;
R₁₅ et R₁₆ sont indépendamment un atome d'hydrogène, un groupe hydroxy, alcanoyloxy inférieur, alcénoyloxy, alcynoyloxy, aryloyloxy ou R₁₅ et R₁₆ forment conjointement un groupe oxo ;
R₁₇ et R₁₈ sont indépendamment un atome d' hydrogène , un groupe alcanoyloxy inférieur, alcénoyloxy, alcynoyloxy, aryloyloxy ou R₁₇ et R₁₈ forment conjointement un groupe oxo ;
R₁₉ et R₂₀ sont indépendamment un atome d'hydrogène, un groupe hydroxy, alcanoyloxy inférieur, alcénoyloxy, alcynoyloxy, aryloyloxy ;
R₂₁ et R₂₂ sont indépendamment un atome d'hydrogène, un groupe alcanoyloxy inférieur, alcénoyloxy, alcynoyloxy, aryloyloxy ou R₂₁ et R₂₂ forment conjointement un groupe oxo ;
R₂₃ et R₂₄ sont indépendamment un atome d'hydrogène, un groupe hydroxy, alcanoyloxy inférieur, alcénoyloxy, alcynoyloxy, aryloyloxy ou
R₂₃ et R₂₄ forment conjointement un groupe oxo ou méthylène ou
R₂₃ et R₂₄ avec l'atome de carbone sur lequel ils sont fixés forment conjointement un noyau oxirane ou
R₂₃ et R₂₄ avec l'atome de carbone sur lequel ils sont fixés forment conjointement un noyau oxétane ;
R₂₅ est un atome d'hydrogène, un groupe hydroxy, alcanoyloxy inférieur, alcénoyloxy, alcynoyloxy, aryloyloxy ;
R₂₆ est un atome d'hydrogène, un groupe hydroxy, alcanoyloxy inférieur, alcénoyloxy, alcynoyloxy, aryloyloxy ; ou
R₂₅ et R₂₆ pris conjointement forment un groupe oxo ; et
R₂₇ est un atome d'hydrogène, un groupe hydroxy, alcanoyloxy inférieur, alcénoyloxy, alcynoyloxy, aryloyloxy.

3. Alcoxyde de métal selon la revendication 2, dans lequel R₂₂ et R₂₃ avec l'atome de carbone sur lequel ils sont fixés forment conjointement un noyau oxétane.

4. Alcoxyde de métal selon la revendication 2 ou la revendication 5,
dans lequel
R₁₅ est -OT₂ ou -OCOCH₃ ;
R₁₆ est un atome d'hydrogène;
R₁₇ et R₁₈ forment conjointement un groupe oxo ;
R₁₉ est -OT₁ ;
R₂₀ et R₂₁ sont un atome d'hydrogène ;
R₂₂ et R₂₃ avec l'atome de carbone sur lequel ils sont fixés forment conjointement un noyau oxétane ;
R₂₄ est -OCOCH₃ ;
R₂₅ est PhCOO- ;
R₂₆ est un atome d'hydrogène;
R₂₇ est un groupe hydroxy ; et
T₁ et T₂ sont indépendamment un atome d'hydrogène ou des groupes hydroxy-protecteur.

5. Alcoxyde de métal selon l'une quelconque des revendications 1 à 4, dans lequel M est un élément du groupe IA, IIA, IIIA, un lanthanide, un actinide ou un métal de transition du groupe IIIA, IVA, VA,ou VIA.

6. Alcoxyde de métal selon l'une quelconque des revendications 1 à 5, dans lequel le métal est le lithium, le magnésium, le sodium, le potassium ou le titane.

7. Alcoxyde de métal selon la revendication 6, dans lequel le métal est le lithium.

## Revendications (Revendications pour l'(les) Etat(s) contractant(s) suivant(s): ES)

1. Procédé de production d'un alcoxyde de métal comprenant la réaction d'un alcool avec un composé organométallique dans lequel l'alcoxyde de métal répond à la formule : 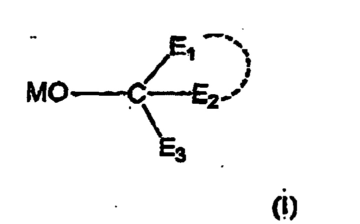 dans laquelle
M est un métal ;
E₁, E₂ et l'atome de carbone sur lequel ils sont fixés comprennent un noyau taxane bi-, tri- ou tétracyclique, et
E₃ est un atome d'hydrogène.

2. Procédé de production d'un alcoxyde de métal comprenant la réaction d'un alcool avec un composé organométallique dans lequel l'alcoxyde de métal répond à la formule : 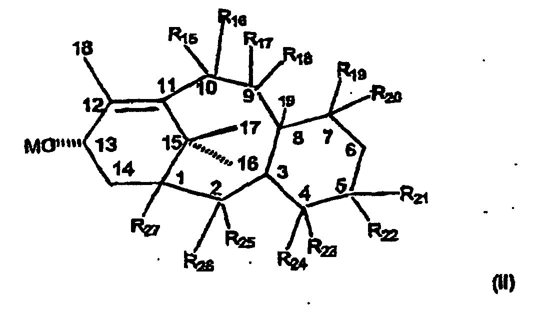 dans laquelle
M est un métal ;
R₁₅ et R₁₆ sont indépendamment un atome d'hydrogène, un groupe hydroxy, alcanoyloxy inférieur, alcénoyloxy, alcynoyloxy, aryloyloxy ou R₁₅ et R₁₆ forment conjointement un groupe oxo ;
R₁₇ et R₁₈ sont indépendamment un atome d'hydrogène, un groupe alcanoyloxy inférieur, alcénoyloxy, alcynoyloxy, aryloyloxy ou R₁₇ et R₁₈ forment conjointement un groupe oxo ;
R₁₉ et R₂₀ sont indépendamment un atome d'hydrogène, un groupe hydroxy, alcanoyloxy inférieur, alcénoyloxy, alcynoyloxy, aryloyloxy;
R₂₁ et R₂₂ sont indépendamment un atome d' hydrogène, un groupe alcanoyloxy inférieur, alcénoyloxy, alcynoyloxy, aryloyloxy ou R₂₁ et R₂₂ forment conjointement un groupe oxo ;
R₂₃ et R₂₄ sont indépendamment un atome d'hydrogène, un groupe hydroxy, alcanoyloxy inférieur, alcénoyloxy, alcynoyloxy, aryloyloxy ou
R₂₃ et R₂₄ forment conjointement un groupe oxo ou méthylène ou
R₂₃ et R₂₄ avec l'atome de carbone sur lequel ils sont fixés forment conjointement un noyau oxirane ou
R₂₃ et R₂₄ avec l'atome de carbone sur lequel ils sont fixés forment conjointement un noyau oxétane ;
R₂₅ est un atome d'hydrogène, un groupe hydroxy, alcanoyloxy inférieur, alcénoyloxy, alcynoyloxy, aryloyloxy ;
R₂₆ est un atome d'hydrogène, un groupe hydroxy, alcanoyloxy inférieur, alcénoyloxy, alcynoyloxy, aryloyloxy ; ou
R₂₅ et R₂₆ pris conjointement forment un groupe oxo ; et
R₂₇ est un atome d'hydrogène, un groupe hydroxy, alcanoyloxy inférieur, alcénoyloxy, alcynoyloxy, aryloyloxy.

3. Procédé selon la revendication 2, dans lequel R₂₂ et R₂₃ avec l'atome de carbone sur lequel ils sont fixés forment conjointement un noyau oxétane.

4. Procédé selon la revendication 2 ou la revendication 5,
dans lequel.
R₁₅ est -OT₂ ou -OCOCH₃ ;
R₁₆ est un atome d'hydrogène;
R₁₇ et R₁₈ forment conjointement un groupe oxo ;
R₁₉ est -OT₁ ;
R₂₀ et R₂₁ sont un atome d'hydrogène;
R₂₂ et R₂₃ avec l'atome de carbone sur lequel ils sont fixés forment conjointement un noyau oxétane ;
R₂₄ est -OCOCH₃;
R₂₅ est PhCOO- ;
R₂₆ est un atome d'hydrogène;
R₂₇ est un groupe hydroxy ; et
T₁ et T₂ sont indépendamment un atome d'hydrogène ou des groupes hydroxy-protecteur.

5. Procédé selon l'une quelconque des revendications 1 à 4, dans lequel M est un élément du groupe IA, IIA, IIIA, un lanthanide, un actinide ou un métal de transition du groupe IIIA, IVA, VA ou VIA.

6. Procédé selon l'une quelconque des revendications 1 à 5, dans lequel le métal est le lithium, le magnésium, le sodium, le potassium ou le titane.

7. Procédé selon la revendication 6, dans lequel le métal est le lithium.
